# EUROPEAN PATENT APPLICATION

(11) **EP 4 582 451 A1**
(43) Date of publication of application: **09.07.2025**
(21) Application number: 23859413.9
(22) Date of filing: 30.08.2023
(51) Int. Cl.: C07K 16/28, C12N 5/24, A61K 39/395, C12N 5/28, A61P 5/08, G01N 33/564

(54) **ANTIBODY BINDING TO THYROID-STIMULATING HORMONE RECEPTOR AND USE THEREOF**

(30) Priority: 31.08.2022 CN 202211054215
(71) Applicant: Changchun Genescience Pharmaceutical Co., Ltd., Changchun, Jilin 130012 (CN)
(72) Inventor: MA, Yanbin, Changchun, Jilin 130012 (CN); WEI, Peipei, Changchun, Jilin 130012 (CN); CHEN, Dakai, Changchun, Jilin 130012 (CN); SUN, Gao, Changchun, Jilin 130012 (CN); KANG, Lishan, Changchun, Jilin 130012 (CN); LUO, Tianhong, Changchun, Jilin 130012 (CN); WANG, Siqin, Changchun, Jilin 130012 (CN); JIN, Lei, Changchun, Jilin 130012 (CN)
(74) Representative: Kolster Oy Ab
(86) International application number: PCT/CN2023/115886
(87) International publication number: WO 2024/046384

(57) **Abstract**

The present invention relates to an antibody specifically binding to a TSHR or an antigen-binding fragment thereof, a pharmaceutical composition comprising the antibody or the antigen-binding fragment thereof, a nucleic acid molecule encoding the antibody or the antigen-binding fragment thereof, a host cell comprising the nucleic acid molecule, and related uses. In addition, the present invention relates to therapeutic and diagnostic use of the antibody or the antigen-binding fragment thereof.

## Description

### BACKGROUND ART

Graves' disease (GD) is a refractory autoimmune disease with symptoms such as an enlarged and overactive thyroid gland, an accelerated heart rate, and eye abnormalities. The disease with abnormally protruding eyes is also called Graves' ophthalmopathy (GO) or Graves' Orbitopathy with Exophthalmos. Based on the understanding of the disease, it is thought to be the result of a complex interaction of genetic and environmental factors. The GO disease not only has a serious impact on the quality of life, but also affects the vigor and work, and the related over-activation of thyroid hormone receptors stimulates the secretion of thyroid hormone, which will lead to an increased risk of death. Currently, main clinical therapies for the GO disease include an antithyroid drug (ATD) therapy, a radioactive iodine (RAI) therapy and a surgical therapy. However, the use of antithyroid drugs is often inefficient and can produce side effects such as bone marrow suppression and liver toxicity. Surgical resection or radioactive iodine ablation also has significant risks and may lead to recurrent nerve damage and hypoparathyroidism after a surgery. In addition, while controlling the thyroid function, it is also necessary to choose a glucocorticoid therapy, a local orbital radiotherapy or a surgical therapy. However, the above therapies are all symptomatic therapies and do not target the pathogenesis of the disease. Currently, there is a lack of effective clinical therapeutic means for the GO disease.

A thyroid-stimulating hormone receptor (TSHR) is mainly expressed on the basolateral surfaces of thyroid follicular cells to induce thyroid cell growth, hormone synthesis and hormone secretion, and is also a main autoantigen of autoimmune thyroid diseases, especially the GD disease. As the course of the disease prolongs, about 25% of GD patients will develop the GO disease.

According to documentary records, some patients with autoimmune thyroid disease (AITD) produce autoantibodies that react with the TSHR (Rees Smith B, et al 1988. Endocrine Reviews 9:106-121). There are two main types of TSHR autoantibodies (TRAbs): a stimulating type and a blocking type. Thyroid-stimulating autoantibodies bind to the TSHR and imitate the effects of TSH, thereby stimulating the thyroid gland to produce high levels of T4 and T3. These autoantibodies are also described as TRAbs with agonist activity (Rees Smith B et al. 2007. Thyroid 17:923 - 938). When these antibodies are present, the feedback control mechanisms of the thyroid function are no longer effective, and the patient develops clinical symptoms of an overactive thyroid characterized by excess thyroid hormone in serum and metabolic consequences thereof. This case is known as the Graves' disease (GD). TRAbs with stimulating activity may also interact with the TSHR in retroorbital tissues to promote the occurrence of the Graves' ophthalmopathy (GO). Based on the current research on the pathogenesis of the GO disease, it is believed that the expression of the TSHR increases in the orbital fibroblasts of GO patients. Moreover, when the signal pathway is activated, it will increase the production of the hyaluronic acid and adipose tissue in the orbit, leading to increased intraocular pressure and exophthalmos, which is an important reason for the GO disease. In addition, more TSHR is expressed in the thyroid gland and testis but less in other tissues, which may have advantages in terms of drug targeting and side effects. Therefore, the TSHR is expected to become a potential target for the treatment of the GO disease.

In addition, an insulin-like growth factor 1 receptor (IGF1R) is considered to play another important role in the pathogenesis of the GO. IGF1R blockers can ameliorate the GO disease. For example, a marketed drug Teprotumumab can competitively inhibit the IGF1R receptor from activating the downstream signal pathway, especially has significant therapeutic effects in ameliorating exophthalmos and reducing inflammations, and has the therapeutic efficiency in the treatment of moderate to severe thyroid eye diseases (TEDs) of up to 78%. It is found that the expression of the TSHR and IGF1R increased in orbital fibroblasts of patients suffering from the GO disease, and there is a certain interaction between the TSHR and the IGF1R. Therefore, the direct blocking of the TSHR (TSAb/antagonist) or the combined therapy with simultaneous antagonism of TSHR and IGF1R may be a way to cure the GD or GO disease.

Currently, there is no drug on the market that targets the TSHR for treating the GO disease, and there is a need in the art to develop therapeutic drugs that target the TSHR.

### TECHNICAL FIELD

The present invention relates to an antibody specifically binding to a TSHR or an antigen-binding fragment thereof, a pharmaceutical composition comprising the antibody or the antigen-binding fragment thereof, a nucleic acid molecule encoding the antibody or the antigen-binding fragment thereof, a host cell comprising the nucleic acid molecule, and related uses. In addition, the present invention relates to therapeutic and diagnostic use of the antibody or the antigen-binding fragment thereof.

### SUMMARY

The inventors of the present application obtain a murine antibody specifically binding to a TSHR through extensive research. The murine antibody has a significant inhibitory effect on TSHR activity, and has cross-binding activity on human/murine/monkey TSHR. In addition, the inventors of the present application further obtain a humanized antibody having TSHR binding activity and inhibitory effect on TSHR activity based on the murine antibody.

Based on this, the present application also provides a pharmaceutical composition comprising the antibody or the antigen-binding fragment thereof, a nucleic acid molecule encoding the antibody or the antigen-binding fragment thereof, a host cell comprising the nucleic acid molecule, and related uses.

### Antibody or antigen-binding fragment thereof

### mAb001 and humanized antibody thereof

Therefore, in a first aspect, the present application provides an antibody capable of specifically binding to a TSHR, or an antigen-binding fragment thereof. The antibody or the antigen-binding fragment thereof comprises:
a VH CDR1 or a variant thereof, a VH CDR2 or a variant thereof, and a VH CDR3 or a variant thereof comprised in a heavy chain variable region (VH) set forth in any one of SEQ ID NOs: 1, 27, 29, and 30; and/or a VL CDR1 or a variant thereof, a VL CDR2 or a variant thereof, and a VL CDR3 or a variant thereof comprised in a light chain variable region (VL) set forth in any one of SEQ ID NOs: 5, 28, and 31;
wherein the variant has one or several amino acid substitutions, deletions or additions (e.g., 1, 2 or 3 amino acid substitutions, deletions or additions, such as conservative substitutions) compared to the sequence from which the variant is derived. In some embodiments, the substitutions are conservative substitutions.

In some embodiments, the antibody or the antigen-binding fragment thereof comprises:
three CDRs comprised in a heavy chain variable region (VH) set forth in any one of SEQ ID NOs: 1, 27, 29, and 30; and/or three CDRs comprised in a light chain variable region (VL) set forth in any one of SEQ ID NOs: 5, 28, and 31.

In some embodiments, the three CDRs comprised in the VH and/or the three CDRs comprised in the VL are defined by the Kabat, IMGT, or Chothia numbering system. In some embodiments, the three CDRs comprised in the VH and/or the three CDRs comprised in the VL are defined by the Kabat numbering system.

In some embodiments, the antibody or the antigen-binding fragment thereof comprises:
a heavy chain variable region (VH) comprising the following three complementary determining regions (CDRs): a VH CDR1 with a sequence set forth in SEQ ID NO: 2, a VH CDR2 with a sequence set forth in SEQ ID NO: 3 or 39, and a VH CDR3 with a sequence set forth in SEQ ID NO: 4; and/or a light chain variable region (VL) comprising the following three complementary determining regions (CDRs): a VL CDR1 with a sequence set forth in SEQ ID NO: 6, a VL CDR2 with a sequence set forth in SEQ ID NO: 7, and a VL CDR3 with a sequence set forth in SEQ ID NO: 8;
wherein the CDR is defined by the Kabat numbering system.

In some embodiments, the antibody or the antigen-binding fragment thereof comprises a framework region sequence derived from a murine immunoglobulin.

In some embodiments, the antibody or the antigen-binding fragment thereof comprises: a heavy chain framework region of a murine heavy chain germline sequence, and/or a light chain framework region of a murine light chain germline sequence.

In some embodiments, the antibody or the antigen-binding fragment thereof comprises:
a heavy chain variable region (VH) comprising a sequence set forth in SEQ ID NO: 1 or a variant thereof; and/or a light chain variable region (VL) comprising a sequence set forth in SEQ ID NO: 5 or a variant thereof;
wherein the variant has one or several amino acid substitutions, deletions or additions (e.g., 1, 2, 3, 4, or 5 amino acid substitutions, deletions or additions), or is a sequence with at least 80%, at least 85%, at least 90%, at least 91%, at least 92%, at least 93%, at least 94%, at least 95%, at least 96%, at least 97%, at least 98%, at least 99%, or 100% sequence identity compared to the sequence from which the variant is derived.

In some embodiments, the substitutions are conservative substitutions.

In some embodiments, the antibody or the antigen-binding fragment thereof comprises: a VH comprising a sequence set forth in SEQ ID NO: 1 and a VL comprising a sequence set forth in SEQ ID NO: 5.

In some embodiments, the antibody or the antigen-binding fragment thereof comprises a framework region sequence derived from a human immunoglobulin.

In some embodiments, the antibody or the antigen-binding fragment thereof comprises a framework region comprised in an amino acid sequence encoded by a human germline antibody gene. In some embodiments, the antibody or the antigen-binding fragment thereof comprises: a heavy chain framework region of a human heavy chain germline sequence, and/or a light chain framework region of a human light chain germline sequence.

In some embodiments, the antibody or the antigen-binding fragment thereof comprises: a heavy chain variable region (VH) comprising a sequence set forth in any one of SEQ ID NOs: 27, 29, and 30 or a variant thereof; and/or a light chain variable region (VL) comprising a sequence set forth in SEQ ID NO: 28 or 31 or a variant thereof;
wherein the variant has one or several amino acid substitutions, deletions or additions (e.g., 1, 2, 3, 4, or 5 amino acid substitutions, deletions or additions), or is a sequence with at least 80%, at least 85%, at least 90%, at least 91%, at least 92%, at least 93%, at least 94%, at least 95%, at least 96%, at least 97%, at least 98%, at least 99%, or 100% sequence identity compared to the sequence from which the variant is derived.

In some embodiments, the substitutions are conservative substitutions.

In some embodiments, the antibody or the antigen-binding fragment thereof comprises: a heavy chain variable region (VH) comprising a sequence set forth in any one of SEQ ID NOs: 27, 29, and 30; and/or a light chain variable region (VL) comprising a sequence set forth in SEQ ID NO: 28 or 31.

In some embodiments, the antibody or the antigen-binding fragment thereof comprises: a VH comprising a sequence set forth in SEQ ID NO: 27 and a VL comprising a sequence set forth in SEQ ID NO: 28.

In some embodiments, the antibody or the antigen-binding fragment thereof comprises: a VH comprising a sequence set forth in SEQ ID NO: 29 and a VL comprising a sequence set forth in SEQ ID NO: 28.

In some embodiments, the antibody or the antigen-binding fragment thereof comprises: a VH comprising a sequence set forth in SEQ ID NO: 30 and a VL comprising a sequence set forth in SEQ ID NO: 28.

In some embodiments, the antibody or the antigen-binding fragment thereof comprises: a VH comprising a sequence set forth in SEQ ID NO: 27 and a VL comprising a sequence set forth in SEQ ID NO: 31.

In some embodiments, the antibody or the antigen-binding fragment thereof comprises: a VH comprising a sequence set forth in SEQ ID NO: 29 and a VL comprising a sequence set forth in SEQ ID NO: 31.

### mAb012 and humanized antibody thereof

Therefore, in a second aspect, the present application provides an antibody capable of specifically binding to TSHR, or an antigen-binding fragment thereof. The antibody or the antigen-binding fragment thereof comprises:
a VH CDR1 or a variant thereof, a VH CDR2 or a variant thereof, and a VH CDR3 or a variant thereof comprised in a heavy chain variable region (VH) set forth in any one of SEQ ID NOs: 9, 32, and 34; and/or a VL CDR1 or a variant thereof, a VL CDR2 or a variant thereof, and a VL CDR3 or a variant thereof comprised in a light chain variable region (VL) set forth in SEQ ID NO: 13 or 33;
wherein the variant has one or several amino acid substitutions, deletions or additions (e.g., 1, 2 or 3 amino acid substitutions, deletions or additions, such as conservative substitutions) compared to the sequence from which the variant is derived. In some embodiments, the substitutions are conservative substitutions.

In some embodiments, the antibody or the antigen-binding fragment thereof comprises:
three CDRs comprised in a heavy chain variable region (VH) set forth in any one of SEQ ID NOs: 9, 32, and 34; and/or three CDRs comprised in a light chain variable region (VL) set forth in SEQ ID NO: 13 or 33.

In some embodiments, the three CDRs comprised in the VH and/or the three CDRs comprised in the VL are defined by the Kabat, IMGT, or Chothia numbering system. In some embodiments, the three CDRs comprised in the VH and/or the three CDRs comprised in the VL are defined by the Kabat numbering system.

In some embodiments, the antibody or the antigen-binding fragment thereof comprises:
a heavy chain variable region (VH) comprising the following three complementary determining regions (CDRs): a VH CDR1 with a sequence set forth in SEQ ID NO: 10, a VH CDR2 with a sequence set forth in SEQ ID NO: 11 or 40, and a VH CDR3 with a sequence set forth in SEQ ID NO: 12 or 41; and/or a light chain variable region (VL) comprising the following three complementary determining regions (CDRs): a VL CDR1 with a sequence set forth in SEQ ID NO: 14, a VL CDR2 with a sequence set forth in SEQ ID NO: 15, and a VL CDR3 with a sequence set forth in SEQ ID NO: 16;
wherein the CDR is defined by the Kabat numbering system.

In some embodiments, the antibody or the antigen-binding fragment thereof comprises:
a heavy chain variable region (VH) comprising the following three complementary determining regions (CDRs): a VH CDR1 with a sequence set forth in SEQ ID NO: 10, a VH CDR2 with a sequence set forth in SEQ ID NO: 11 or 40, and a VH CDR3 with a sequence set forth in SEQ ID NO: 12; and/or a light chain variable region (VL) comprising the following three complementary determining regions (CDRs): a VL CDR1 with a sequence set forth in SEQ ID NO: 14, a VL CDR2 with a sequence set forth in SEQ ID NO: 15, and a VL CDR3 with a sequence set forth in SEQ ID NO: 16;
wherein the CDR is defined by the Kabat numbering system.

In some embodiments, the antibody or the antigen-binding fragment thereof comprises:
a heavy chain variable region (VH) comprising the following three complementary determining regions (CDRs): a VH CDR1 with a sequence set forth in SEQ ID NO: 10, a VH CDR2 with a sequence set forth in SEQ ID NO: 11 or 40, and a VH CDR3 with a sequence set forth in SEQ ID NO: 41; and/or a light chain variable region (VL) comprising the following three complementary determining regions (CDRs): a VL CDR1 with a sequence set forth in SEQ ID NO: 14, a VL CDR2 with a sequence set forth in SEQ ID NO: 15, and a VL CDR3 with a sequence set forth in SEQ ID NO: 16;
wherein the CDR is defined by the Kabat numbering system.

In some embodiments, the antibody or the antigen-binding fragment thereof comprises a framework region sequence derived from a murine immunoglobulin.

In some embodiments, the antibody or the antigen-binding fragment thereof comprises: a heavy chain framework region of a murine heavy chain germline sequence, and/or a light chain framework region of a murine light chain germline sequence.

In some embodiments, the antibody or the antigen-binding fragment thereof comprises:
a heavy chain variable region (VH) comprising a sequence set forth in SEQ ID NO: 9 or a variant thereof; and/or a light chain variable region (VL) comprising a sequence set forth in SEQ ID NO: 13 or a variant thereof;
wherein the variant has one or several amino acid substitutions, deletions or additions (e.g., 1, 2, 3, 4, or 5 amino acid substitutions, deletions or additions), or is a sequence with at least 80%, at least 85%, at least 90%, at least 91%, at least 92%, at least 93%, at least 94%, at least 95%, at least 96%, at least 97%, at least 98%, at least 99%, or 100% sequence identity compared to the sequence from which the variant is derived.

In some embodiments, the substitutions are conservative substitutions.

In some embodiments, the antibody or the antigen-binding fragment thereof comprises: a VH comprising a sequence set forth in SEQ ID NO: 9 and a VL comprising a sequence set forth in SEQ ID NO: 13.

In some embodiments, the antibody or the antigen-binding fragment thereof comprises a framework region sequence derived from a human immunoglobulin.

In some embodiments, the antibody or the antigen-binding fragment thereof comprises a framework region comprised in an amino acid sequence encoded by a human germline antibody gene. In some embodiments, the antibody or the antigen-binding fragment thereof comprises: a heavy chain framework region of a human heavy chain germline sequence, and/or a light chain framework region of a human light chain germline sequence.

In some embodiments, the antibody or the antigen-binding fragment thereof comprises: a heavy chain variable region (VH) comprising a sequence set forth in SEQ ID NO: 32 or 34 or a variant thereof; and/or a light chain variable region (VL) comprising a sequence set forth in SEQ ID NO: 33 or a variant thereof;
wherein the variant has one or several amino acid substitutions, deletions or additions (e.g., 1, 2, 3, 4, or 5 amino acid substitutions, deletions or additions), or is a sequence with at least 80%, at least 85%, at least 90%, at least 91%, at least 92%, at least 93%, at least 94%, at least 95%, at least 96%, at least 97%, at least 98%, at least 99%, or 100% sequence identity compared to the sequence from which the variant is derived.

In some embodiments, the substitutions are conservative substitutions.

In some embodiments, the antibody or the antigen-binding fragment thereof comprises: a heavy chain variable region (VH) comprising a sequence set forth in SEQ ID NO: 32 or 34; and/or a light chain variable region (VL) comprising a sequence set forth in SEQ ID NO: 33.

In some embodiments, the antibody or the antigen-binding fragment thereof comprises: a VH comprising a sequence set forth in SEQ ID NO: 32 and a VL comprising a sequence set forth in SEQ ID NO: 33.

In some embodiments, the antibody or the antigen-binding fragment thereof comprises: a VH comprising a sequence set forth in SEQ ID NO: 34 and a VL comprising a sequence set forth in SEQ ID NO: 33.

### mAb021 and humanized antibody thereof

Therefore, in a third aspect, the present application provides an antibody capable of specifically binding to TSHR, or an antigen-binding fragment thereof. The antibody or the antigen-binding fragment thereof comprises:
a VH CDR1 or a variant thereof, a VH CDR2 or a variant thereof, and a VH CDR3 or a variant thereof comprised in a heavy chain variable region (VH) set forth in any one of SEQ ID NOs: 17, 35, and 37; and/or a VL CDR1 or a variant thereof, a VL CDR2 or a variant thereof, and a VL CDR3 or a variant thereof comprised in a light chain variable region (VL) set forth in any one of SEQ ID NOs: 21, 36, and 38;
wherein the variant has one or several amino acid substitutions, deletions or additions (e.g., 1, 2 or 3 amino acid substitutions, deletions or additions, such as conservative substitutions) compared to the sequence from which the variant is derived. In some embodiments, the substitutions are conservative substitutions.

In some embodiments, the antibody or the antigen-binding fragment thereof comprises:
three CDRs comprised in a heavy chain variable region (VH) set forth in any one of SEQ ID NOs: 17, 35, and 37; and/or three CDRs comprised in a light chain variable region (VL) set forth in any one of SEQ ID NOs: 21, 36, and 38.

In some embodiments, the three CDRs comprised in the VH and/or the three CDRs comprised in the VL are defined by the Kabat, IMGT, or Chothia numbering system. In some embodiments, the three CDRs comprised in the VH and/or the three CDRs comprised in the VL are defined by the Kabat numbering system.

In some embodiments, the antibody or the antigen-binding fragment thereof comprises:
a heavy chain variable region (VH) comprising the following three complementary determining regions (CDRs): a VH CDR1 with a sequence set forth in SEQ ID NO: 18, a VH CDR2 with a sequence set forth in SEQ ID NO: 19, and a VH CDR3 with a sequence set forth in SEQ ID NO: 20; and/or a light chain variable region (VL) comprising the following three complementary determining regions (CDRs): a VL CDR1 with a sequence set forth in SEQ ID NO: 22, a VL CDR2 with a sequence set forth in SEQ ID NO: 23, and a VL CDR3 with a sequence set forth in SEQ ID NO: 24;
wherein the CDR is defined by the Kabat numbering system.

In some embodiments, the antibody or the antigen-binding fragment thereof comprises a framework region sequence derived from a murine immunoglobulin.

In some embodiments, the antibody or the antigen-binding fragment thereof comprises: a heavy chain framework region of a murine heavy chain germline sequence, and/or a light chain framework region of a murine light chain germline sequence.

In some embodiments, the antibody or the antigen-binding fragment thereof comprises:
a heavy chain variable region (VH) comprising a sequence set forth in SEQ ID NO: 17 or a variant thereof; and/or a light chain variable region (VL) comprising a sequence set forth in SEQ ID NO: 21 or a variant thereof;
wherein the variant has one or several amino acid substitutions, deletions or additions (e.g., 1, 2, 3, 4, or 5 amino acid substitutions, deletions or additions), or is a sequence with at least 80%, at least 85%, at least 90%, at least 91%, at least 92%, at least 93%, at least 94%, at least 95%, at least 96%, at least 97%, at least 98%, at least 99%, or 100% sequence identity compared to the sequence from which the variant is derived.

In some embodiments, the substitutions are conservative substitutions.

In some embodiments, the antibody or the antigen-binding fragment thereof comprises: a VH comprising a sequence set forth in SEQ ID NO: 17 and a VL comprising a sequence set forth in SEQ ID NO: 21.

In some embodiments, the antibody or the antigen-binding fragment thereof comprises a framework region sequence derived from a human immunoglobulin.

In some embodiments, the antibody or the antigen-binding fragment thereof comprises a framework region comprised in an amino acid sequence encoded by a human germline antibody gene. In some embodiments, the antibody or the antigen-binding fragment thereof comprises: a heavy chain framework region of a human heavy chain germline sequence, and/or a light chain framework region of a human light chain germline sequence.

In some embodiments, the antibody or the antigen-binding fragment thereof comprises: a heavy chain variable region (VH) comprising a sequence set forth in SEQ ID NO: 35 or 37 or a variant thereof; and/or a light chain variable region (VL) comprising a sequence set forth in SEQ ID NO: 36 or 38 or a variant thereof;
wherein the variant has one or several amino acid substitutions, deletions or additions (e.g., 1, 2, 3, 4, or 5 amino acid substitutions, deletions or additions), or is a sequence with at least 80%, at least 85%, at least 90%, at least 91%, at least 92%, at least 93%, at least 94%, at least 95%, at least 96%, at least 97%, at least 98%, at least 99%, or 100% sequence identity compared to the sequence from which the variant is derived.

In some embodiments, the substitutions are conservative substitutions.

In some embodiments, the antibody or the antigen-binding fragment thereof comprises: a heavy chain variable region (VH) comprising a sequence set forth in SEQ ID NO: 35 or 37; and/or a light chain variable region (VL) comprising a sequence set forth in SEQ ID NO: 36 or 38.

In some embodiments, the antibody or the antigen-binding fragment thereof comprises: a VH comprising a sequence set forth in SEQ ID NO: 35 and a VL comprising a sequence set forth in SEQ ID NO: 36.

In some embodiments, the antibody or the antigen-binding fragment thereof comprises: a VH comprising a sequence set forth in SEQ ID NO: 37 and a VL comprising a sequence set forth in SEQ ID NO: 38.

In some embodiments, in the first aspect, the second aspect or the third aspect, the antibody or the antigen-binding fragment thereof further comprises a constant region derived from a mammalian (e.g., human or murine) immunoglobulin.

In some embodiments, a heavy chain of the antibody or the antigen-binding fragment thereof comprises a heavy chain constant region derived from a human immunoglobulin (e.g., IgG1, IgG2, IgG3, or IgG4), and/or a light chain of the antibody or the antigen-binding fragment thereof comprises a light chain constant region derived from a human immunoglobulin (e.g., κ or λ).

In some embodiments, a heavy chain of the antibody or the antigen-binding fragment thereof comprises a heavy chain constant region (CH) of a human immunoglobulin or a variant thereof, and the variant has one or more amino acid substitutions, deletions or additions (e.g., at most 20, at most 15, at most 10, or at most 5 amino acid substitutions, deletions or additions, such as 1, 2, 3, 4, or 5 amino acid substitutions, deletions or additions) compared to the sequence from which the variant is derived; and/or,
a light chain of the antibody or the antigen-binding fragment thereof comprises a light chain constant region (CL) of a human immunoglobulin or a variant thereof, and the variant has one or more amino acid substitutions, deletions or additions (e.g., at most 20, at most 15, at most 10, or at most 5 amino acid substitutions, deletions or additions, such as 1, 2, 3, 4, or 5 amino acid substitutions, deletions or additions) compared to the sequence from which the variant is derived. The substitutions may be conservative substitutions or non-conservative substitutions.

In some embodiments, the antibody or the antigen-binding fragment thereof comprises a heavy chain constant region (CH) of a wild type human immunoglobulin.

In some other embodiments, the antibody or the antigen-binding fragment thereof comprises a variant of a heavy chain constant region (CH) of a human immunoglobulin, and the variant of the heavy chain constant region (CH) may have the same or basically the same properties as a wild type sequence from which the variant is derived. In some embodiments, the variant of the heavy chain constant region (CH) may have one or more amino acid conservative substitutions compared to the sequence from which the variant is derived.

In some other embodiments, the antibody or the antigen-binding fragment thereof comprises a variant of a heavy chain constant region (CH) of a human immunoglobulin, and the variant of the heavy chain constant region (CH) may comprise one or more amino acid mutations or chemical modifications to change one or more of the following properties of the antibody of the present invention: Fc receptor binding, antibody glycosylation, the number of cysteine residues, effector cell functions or complement functions, etc. Function changes can be produced by replacing at least one amino acid residue in a constant region of an antibody with a different residue or by chemical modification. For example, the affinity of the antibody for an effector ligand (e.g., FcR or complement C1q) is changed, thereby changing effector functions (e.g., reducing or enhancing). An Fc region of an antibody mediates several important effector functions, such as ADCC, phagocytosis, CDC, etc.

In some embodiments, the antibody or the antigen-binding fragment thereof of the present invention comprises a variant of a heavy chain constant region (CH) of a human immunoglobulin, and the variant may have reduced or eliminated effector functions compared to a wild type sequence from which the variant is derived.

In some embodiments, the antibody or the antigen-binding fragment thereof of the present invention comprises a variant of a heavy chain constant region (CH) of a human immunoglobulin, and the variant may have enhanced effector functions compared to a wild type sequence from which the variant is derived.

In some embodiments, the antibody or the antigen-binding fragment thereof of the present invention comprises a heavy chain constant region (CH) of a human immunoglobulin or a variant thereof, and the variant may have the same or basically the same effector functions as a wild type sequence from which the variant is derived.

In some embodiments, the antibody or the antigen-binding fragment thereof of the present invention comprises a variant of a heavy chain constant region (CH) of a human immunoglobulin, and the variant has (i) prolonged half-life, (ii) enhanced efficacy, and/or (iii) reduced ADCC activity compared to a wild type sequence from which the variant is derived.

In some embodiments, the heavy chain constant region (CH) is a heavy chain constant region of an IgG, such as a heavy chain constant region of IgG1, IgG2, IgG3, or IgG4.

In some embodiments, the light chain constant region is a κ light chain constant region or a λ light chain constant region.

In some embodiments, a heavy chain of the antibody or the antigen-binding fragment thereof comprises a heavy chain constant region derived from a murine immunoglobulin (e.g., IgG1, IgG2, IgG3, or IgG4), and a light chain of the antibody or the antigen-binding fragment thereof comprises a light chain constant region derived from a murine immunoglobulin (e.g., κ or λ).

In some embodiments, in the first aspect, the second aspect or the third aspect, a heavy chain of the antibody or the antigen-binding fragment thereof comprises a heavy chain constant region derived from a human immunoglobulin IgG4, and/or a light chain of the antibody or the antigen-binding fragment thereof comprises a light chain constant region derived from a human immunoglobulin (e.g., κ or λ).

In some embodiments, compared to the heavy chain constant region of a wild type human immunoglobulin IgG4, the heavy chain constant region derived from the human immunoglobulin IgG4 comprises substitution mutations S228P, L235E, M252Y, S254T, and/or T256E (e.g., (i) S228P, (ii) L235E, and/or (iii) M252Y, S254T and T256E).

In some embodiments, compared to the heavy chain constant region of a wild type human immunoglobulin IgG4, the heavy chain constant region derived from the human immunoglobulin IgG4 comprises a substitution mutation S228P; in some embodiments, the heavy chain constant region derived from the human immunoglobulin IgG4 further comprises substitution mutations M252Y, S254T and T256E; and in some embodiments, the heavy chain constant region derived from the human immunoglobulin IgG4 further comprises substitution mutations L235E, M252Y, S254T and T256E.

In some embodiments, the amino acid position of the substitution mutation is defined by the EU numbering system.

In some embodiments, a heavy chain of the antibody or the antigen-binding fragment thereof comprises a heavy chain constant region (CH) set forth in any one of SEQ ID NOs: 25 and 42-43, and/or a light chain of the antibody or the antigen-binding fragment thereof comprises a light chain constant region (CL) set forth in SEQ ID NO: 26.

In some embodiments, the antibody or the antigen-binding fragment thereof comprises:
(1) a heavy chain comprising a VH with a sequence set forth in SEQ ID NO: 27 and a CH with a sequence set forth in any one of SEQ ID NOs: 25 and 42-43, and/or a light chain comprising a VL with a sequence set forth in SEQ ID NO: 28 and a CL with a sequence set forth in SEQ ID NO: 26;
(2) a heavy chain comprising a VH with a sequence set forth in SEQ ID NO: 29 and a CH with a sequence set forth in any one of SEQ ID NOs: 25 and 42-43, and/or a light chain comprising a VL with a sequence set forth in SEQ ID NO: 28 and a CL with a sequence set forth in SEQ ID NO: 26;
(3) a heavy chain comprising a VH with a sequence set forth in SEQ ID NO: 30 and a CH with a sequence set forth in any one of SEQ ID NOs: 25 and 42-43, and/or a light chain comprising a VL with a sequence set forth in SEQ ID NO: 28 and a CL with a sequence set forth in SEQ ID NO: 26;
(4) a heavy chain comprising a VH with a sequence set forth in SEQ ID NO: 27 and a CH with a sequence set forth in any one of SEQ ID NOs: 25 and 42-43, and/or a light chain comprising a VL with a sequence set forth in SEQ ID NO: 31 and a CL with a sequence set forth in SEQ ID NO: 26;
(5) a heavy chain comprising a VH with a sequence set forth in SEQ ID NO: 29 and a CH with a sequence set forth in any one of SEQ ID NOs: 25 and 42-43, and/or a light chain comprising a VL with a sequence set forth in SEQ ID NO: 31 and a CL with a sequence set forth in SEQ ID NO: 26;
(6) a heavy chain comprising a VH with a sequence set forth in SEQ ID NO: 32 and a CH with a sequence set forth in any one of SEQ ID NOs: 25 and 42-43, and/or a light chain comprising a VL with a sequence set forth in SEQ ID NO: 33 and a CL with a sequence set forth in SEQ ID NO: 26;
(7) a heavy chain comprising a VH with a sequence set forth in SEQ ID NO: 34 and a CH with a sequence set forth in any one of SEQ ID NOs: 25 and 42-43, and/or a light chain comprising a VL with a sequence set forth in SEQ ID NO: 33 and a CL with a sequence set forth in SEQ ID NO: 26;
(8) a heavy chain comprising a VH with a sequence set forth in SEQ ID NO: 35 and a CH with a sequence set forth in any one of SEQ ID NOs: 25 and 42-43, and/or a light chain comprising a VL with a sequence set forth in SEQ ID NO: 36 and a CL with a sequence set forth in SEQ ID NO: 26; or,
(9) a heavy chain comprising a VH with a sequence set forth in SEQ ID NO: 37 and a CH with a sequence set forth in any one of SEQ ID NOs: 25 and 42-43, and/or a light chain comprising a VL with a sequence set forth in SEQ ID NO: 38 and a CL with a sequence set forth in SEQ ID NO: 26.

In some embodiments, the antibody or the antigen-binding fragment thereof in the first aspect comprises:
(1) a heavy chain comprising a sequence set forth in SEQ ID NO: 46, and/or a light chain comprising a sequence set forth in SEQ ID NO: 51;
(2) a heavy chain comprising a sequence set forth in SEQ ID NO: 47, and/or a light chain comprising a sequence set forth in SEQ ID NO: 51; or,
(3) a heavy chain comprising a sequence set forth in SEQ ID NO: 48, and/or a light chain comprising a sequence set forth in SEQ ID NO: 51.

In some embodiments, in the first aspect, the second aspect or the third aspect, a heavy chain of the antibody or the antigen-binding fragment thereof comprises a heavy chain constant region derived from a human immunoglobulin IgG1, and/or a light chain of the antibody or the antigen-binding fragment thereof comprises a light chain constant region derived from a human immunoglobulin (e.g., κ or λ).

In some embodiments, compared to the heavy chain constant region of a wild type human immunoglobulin IgG1, the heavy chain constant region derived from the human immunoglobulin IgG1 comprises substitution mutations L234A, L235A, M252Y, S254T, and/or T256E (e.g., (i) L234A and L235A, and/or (ii) M252Y, S254T and T256E).

In some embodiments, compared to the heavy chain constant region of a wild type human immunoglobulin IgG1, the heavy chain constant region derived from the human immunoglobulin IgG1 comprises substitution mutations L234A and L235A; and in some embodiments, the heavy chain constant region derived from the human immunoglobulin IgG1 further comprises substitution mutations M252Y, S254T and T256E.

In some embodiments, the amino acid position of the substitution mutation is defined by the EU numbering system.

In some embodiments, a heavy chain of the antibody or the antigen-binding fragment thereof comprises a heavy chain constant region (CH) set forth in SEQ ID NO: 44 or 45, and/or a light chain of the antibody or the antigen-binding fragment thereof comprises a light chain constant region (CL) set forth in SEQ ID NO: 26.

In some embodiments, the antibody or the antigen-binding fragment thereof comprises:
(1) a heavy chain comprising a VH with a sequence set forth in SEQ ID NO: 27 and a CH with a sequence set forth in SEQ ID NO: 44 or 45, and/or a light chain comprising a VL with a sequence set forth in SEQ ID NO: 28 and a CL with a sequence set forth in SEQ ID NO: 26;
(2) a heavy chain comprising a VH with a sequence set forth in SEQ ID NO: 29 and a CH with a sequence set forth in SEQ ID NO: 44 or 45, and/or a light chain comprising a VL with a sequence set forth in SEQ ID NO: 28 and a CL with a sequence set forth in SEQ ID NO: 26;
(3) a heavy chain comprising a VH with a sequence set forth in SEQ ID NO: 30 and a CH with a sequence set forth in SEQ ID NO: 44 or 45, and/or a light chain comprising a VL with a sequence set forth in SEQ ID NO: 28 and a CL with a sequence set forth in SEQ ID NO: 26;
(4) a heavy chain comprising a VH with a sequence set forth in SEQ ID NO: 27 and a CH with a sequence set forth in SEQ ID NO: 44 or 45, and/or a light chain comprising a VL with a sequence set forth in SEQ ID NO: 31 and a CL with a sequence set forth in SEQ ID NO: 26;
(5) a heavy chain comprising a VH with a sequence set forth in SEQ ID NO: 29 and a CH with a sequence set forth in SEQ ID NO: 44 or 45, and/or a light chain comprising a VL with a sequence set forth in SEQ ID NO: 31 and a CL with a sequence set forth in SEQ ID NO: 26;
(6) a heavy chain comprising a VH with a sequence set forth in SEQ ID NO: 32 and a CH with a sequence set forth in SEQ ID NO: 44 or 45, and/or a light chain comprising a VL with a sequence set forth in SEQ ID NO: 33 and a CL with a sequence set forth in SEQ ID NO: 26;
(7) a heavy chain comprising a VH with a sequence set forth in SEQ ID NO: 34 and a CH with a sequence set forth in SEQ ID NO: 44 or 45, and/or a light chain comprising a VL with a sequence set forth in SEQ ID NO: 33 and a CL with a sequence set forth in SEQ ID NO: 26;
(8) a heavy chain comprising a VH with a sequence set forth in SEQ ID NO: 35 and a CH with a sequence set forth in SEQ ID NO: 44 or 45, and/or a light chain comprising a VL with a sequence set forth in SEQ ID NO: 36 and a CL with a sequence set forth in SEQ ID NO: 26; or,
(9) a heavy chain comprising a VH with a sequence set forth in SEQ ID NO: 37 and a CH with a sequence set forth in SEQ ID NO: 44 or 45, and/or a light chain comprising a VL with a sequence set forth in SEQ ID NO: 38 and a CL with a sequence set forth in SEQ ID NO: 26.

In some embodiments, the antibody or the antigen-binding fragment thereof in the first aspect comprises:
(1) a heavy chain comprising a sequence set forth in SEQ ID NO: 49, and/or a light chain comprising a sequence set forth in SEQ ID NO: 51; or,
(2) a heavy chain comprising a sequence set forth in SEQ ID NO: 50, and/or a light chain comprising a sequence set forth in SEQ ID NO: 51.

In some embodiments, in the first aspect, the second aspect or the third aspect, the antigen-binding fragment is selected from Fab, Fab', (Fab')₂, Fd, Fv, disulfide-linked Fv, scFv, di-scFv, (scFv)₂, a diabody, and a single domain antibody (sdAb); and/or the antibody is a murine antibody, a humanized antibody, a chimeric antibody, a bispecific antibody, or a multispecific antibody.

In a fourth aspect, the present application also provides an isolated nucleic acid molecule encoding the antibody or the antigen-binding fragment thereof as described in the first aspect, the second aspect or the third aspect, or the heavy chain variable region and/or the light chain variable region thereof, or the heavy chain and/or the light chain thereof.

In some embodiments, the isolated nucleic acid molecule comprises a first nucleotide sequence encoding the heavy chain or the heavy chain variable region of the antibody or the antigen-binding fragment thereof of the present invention and a second nucleotide sequence encoding the light chain or the light chain variable region of the antibody or the antigen-binding fragment thereof, wherein the first nucleotide sequence and the second nucleotide sequence are present on the same or different isolated nucleic acid molecules. When the first nucleotide sequence and the second nucleotide sequence are present on different isolated nucleic acid molecules, the isolated nucleic acid molecule of the present invention comprises a first nucleic acid molecule comprising the first nucleotide sequence and a second nucleic acid molecule comprising the second nucleotide sequence.

In a fifth aspect, the present application also provides a vector comprising the isolated nucleic acid molecule as described above. In some embodiments, the vector is a cloning vector or an expression vector.

In some embodiments, the vector comprises a first nucleotide sequence encoding the heavy chain or the heavy chain variable region of the antibody or the antigen-binding fragment thereof of the present invention and a second nucleotide sequence encoding the light chain or the light chain variable region of the antibody or the antigen-binding fragment thereof, wherein the first nucleotide sequence and the second nucleotide sequence are present on the same or different vectors. When the first nucleotide sequence and the second nucleotide sequence are present on different vectors, the vector of the present invention comprises a first vector comprising the first nucleotide sequence and a second vector comprising the second nucleotide sequence.

In a sixth aspect, the present application also provides a host cell comprising the isolated nucleic acid molecule or the vector as described above.

Such host cells include, but are not limited to, prokaryotic cells such as bacterial cells (e.g., Escherichia coli cells), and eukaryotic cells such as fungal cells (e.g., yeast cells), insect cells, plant cells, and animal cells (e.g., mammalian cells, such as mouse cells and human cells). In some embodiments, the host cell is a microorganism.

The antibody of the present invention can be prepared by various methods known in the art, for example, by a genetic engineering recombination technology. For example, DNA molecules encoding heavy chain and light chain genes of the antibody of the present invention are obtained by chemical synthesis or PCR amplification. The obtained DNA molecules are inserted into an expression vector which is then used for transfecting host cells. Then, the transfected host cells are cultured under specific conditions to express the antibody of the present invention.

The antigen-binding fragments of the present invention can be obtained by hydrolyzing intact antibody molecules (see Morimoto et al., J. Biochem. Biophys. Methods 24:107-117 (1992) and Brennan et al., Science 229:81 (1985)). In addition, these antigen-binding fragments can also be produced directly from recombinant host cells (reviewed in Hudson, Curr. Opin. Immunol. 11: 548-557 (1999); Little et al., Immunol. Today, 21: 364-370 (2000)). For example, Fab' fragments can be obtained directly from host cells. Fab' fragments can be chemically coupled to form F(ab')₂ fragments (Carter et al., Bio/Technology, 10: 163-167 (1992)). In addition, Fv, Fab or F(ab')₂ fragments can also be directly isolated from a culture fluid of recombinant host cells. Other technologies for preparing these antigen-binding fragments are well known to those of ordinary skill in the art.

In a seventh aspect, the present application also provides a method for preparing the antibody or the antigen-binding fragment thereof as described in the first aspect, the second aspect or the third aspect. The method comprises the steps of culturing the host cell as described above under the conditions allowing the expression of the antibody or the antigen-binding fragment thereof, and recovering the antibody or the antigen-binding fragment thereof from the cultured host cell culture.

### Therapeutic uses

In an eighth aspect, the present application also provides a bispecific or multispecific molecule which comprises the antibody or the antigen-binding fragment thereof as described in the first aspect, the second aspect or the third aspect.

In some embodiments, the bispecific or multispecific molecule specifically binds to TSHR, and additionally specifically binds to one or more other targets.

In some embodiments, the bispecific or multispecific molecule further comprises at least one molecule with a second binding specificity for a second target (e.g., a second antibody).

In some embodiments, the bispecific or multispecific molecule further comprises at least one second antibody that specifically binds to IGF1R. In some embodiments, the second antibody has antagonism on IGF1R signaling.

In a ninth aspect, the present application also provides an immunoconjugate which comprises the antibody or the antigen-binding fragment thereof as described in the first aspect, the second aspect or the third aspect and a therapeutic agent linked to the antibody or the antigen-binding fragment thereof.

In some embodiments, the therapeutic agent is selected from an IGF1R antagonist or inhibitor.

In some embodiments, the immunoconjugate is an antibody-drug conjugate (ADC).

In a tenth aspect, the present application also provides a pharmaceutical composition which comprises the antibody or the antigen-binding fragment thereof as described in the first aspect, the second aspect or the third aspect, the isolated nucleic acid molecule as described in the fourth aspect, the vector as described in the fifth aspect, the host cell as described in the sixth aspect, the bispecific or multispecific molecule as described in the eighth aspect, or the immunoconjugate as described in the ninth aspect, as well as a pharmaceutically acceptable carrier and/or excipient.

In some embodiments, the pharmaceutical composition further comprises another pharmaceutically active agent, such as another drug for treating an autoimmune thyroid disease (e.g., Graves' disease or Graves' ophthalmopathy) or thyroid cancer.

In some embodiments, the pharmaceutical composition further comprises an IGF1R antagonist or inhibitor, and/or another TSHR antagonist or inhibitor.

In some exemplary embodiments, the pharmaceutically acceptable carrier and/or excipient comprises sterile injectable liquid (e.g., an aqueous or non-aqueous suspension or solution). In some exemplary embodiments, such sterile injectable liquid is selected from water for injection (WFI), bacteriostatic water for injection (BWFI), a sodium chloride solution (e.g., 0.9% NaCl), a glucose solution (e.g., 5% glucose), a solution comprising a surfactant (e.g., 0.01% polysorbate 20), a pH buffer (e.g., phosphate buffer), a Ringer's solution, and any combination thereof.

In an eleventh aspect, the present application also provides the use of the antibody or the antigen-binding fragment thereof as described in the first aspect, the second aspect or the third aspect, the isolated nucleic acid molecule as described in the fourth aspect, the vector as described in the fifth aspect, the host cell as described in the sixth aspect, the bispecific or multispecific molecule as described in the eighth aspect, the immunoconjugate as described in the ninth aspect, or the pharmaceutical composition as described in the tenth aspect for preparing a drug. The drug is used for preventing and/or treating a TSHR-related disease in a subject.

In some embodiments, the TSHR-related disease benefits from the antagonism on TSHR signaling.

In some embodiments, the TSHR-related disease is an autoimmune thyroid disease (e.g., Graves' disease or Graves' ophthalmopathy), thyroid cancer, or a combination thereof.

In some embodiments, the subject is a mammal, such as a human, murine, or monkey.

In some embodiments, the antibody or the antigen-binding fragment thereof, the isolated nucleic acid molecule, the vector, the host cell, the bispecific or multispecific molecule, the immunoconjugate, or the pharmaceutical composition is used alone or in combination with another pharmaceutically active agent (e.g., another drug for treating an autoimmune thyroid disease (e.g., Graves' disease or Graves' ophthalmopathy) or thyroid cancer), for example, administered simultaneously or sequentially.

In some embodiments, the antibody or the antigen-binding fragment thereof, the isolated nucleic acid molecule, the vector, the host cell, the bispecific or multispecific molecule, the immunoconjugate, or the pharmaceutical composition is used in combination with an IGF1R antagonist or inhibitor and/or another TSHR antagonist or inhibitor, for example, administered simultaneously or sequentially.

In a twelfth aspect, the present application also provides a method for preventing and/or treating a TSHR-related disease in a subject, comprising: administering to a subject in need thereof an effective amount of the antibody or the antigen-binding fragment thereof as described in the first aspect, the second aspect or the third aspect, the isolated nucleic acid molecule as described in the fourth aspect, the vector as described in the fifth aspect, the host cell as described in the sixth aspect, the bispecific or multispecific molecule as described in the eighth aspect, the immunoconjugate as described in the ninth aspect, or the pharmaceutical composition as described in the tenth aspect.

In some embodiments, the TSHR-related disease benefits from the antagonism on TSHR signaling.

In some embodiments, the TSHR-related disease is an autoimmune thyroid disease (e.g., Graves' disease or Graves' ophthalmopathy), thyroid cancer, or a combination thereof.

In some embodiments, the subject is a mammal, such as a human, murine, or monkey.

In some embodiments, the antibody or the antigen-binding fragment thereof as described in the first aspect, the second aspect or the third aspect, the isolated nucleic acid molecule as described in the fourth aspect, the vector as described in the fifth aspect, the host cell as described in the sixth aspect, the bispecific or multispecific molecule as described in the eighth aspect, the immunoconjugate as described in the ninth aspect, or the pharmaceutical composition as described in the tenth aspect can be administered in combination with another pharmaceutically active agent (e.g., another drug for treating an autoimmune thyroid disease (e.g., Graves' disease or Graves' ophthalmopathy) or thyroid cancer), for example, administered simultaneously or sequentially.

In some embodiments, the antibody or the antigen-binding fragment thereof as described in the first aspect, the second aspect or the third aspect, the isolated nucleic acid molecule as described in the fourth aspect, the vector as described in the fifth aspect, the host cell as described in the sixth aspect, the bispecific or multispecific molecule as described in the eighth aspect, the immunoconjugate as described in the ninth aspect, or the pharmaceutical composition as described in the tenth aspect can be administered in combination with an IGF1R antagonist or inhibitor and/or another TSHR antagonist or inhibitor, for example, administered simultaneously or sequentially.

The antibody or the antigen-binding fragment thereof or the pharmaceutical composition of the present application can be formulated into any dosage form known in the medical arts, for example, tablets, pills, suspensions, emulsions, solutions, gels, capsules, powders, granules, elixirs, lozenges, suppositories, injections (including injection solutions, sterile powders for injection, and concentrated solutions for injection), inhalants, sprays, etc. The preferred dosage form depends on the intended mode of administration and therapeutic use. The antibody or the antigen-binding fragment thereof or the pharmaceutical composition of the present invention should be sterile and stable under the conditions of production and storage. A preferred dosage form is an injection. Such injections may be sterile injection solutions. For example, the sterile injection solutions can be prepared by incorporating a necessary dose of the antibody or the antigen-binding fragment thereof of the present invention in an appropriate solvent, and optionally, incorporating other desired ingredients (including but not limited to pH adjusting agents, surfactants, adjuvants, ionic strength enhancers, isotonic agents, preservatives, diluents, or any combination thereof), followed by performing filtration sterilization. Additionally, the sterile injectable solutions can be prepared as sterile lyophilized powders (e.g., by vacuum drying or freeze drying) for ease of storage and use. Such sterile lyophilized powders can be dispersed in suitable carriers before use, such as water for injection (WFI), bacteriostatic water for injection (BWFI), a sodium chloride solution (e.g., 0.9% NaCl), a glucose solution (e.g., 5% glucose), a solution comprising a surfactant (e.g., 0.01% polysorbate 20), a pH buffer (e.g., phosphate buffer), a Ringer's solution, and any combination thereof.

The antibody or the antigen-binding fragment thereof or the pharmaceutical composition of the present application can be administered by any suitable method known in the art, including but not limited to, oral, buccal, sublingual, ocular, topical, parenteral, rectal, intrathecal, intracytoplasmic reticulum, inguinal, intravesical, topical (e.g., powders, ointments or drops), or nasal routes. However, for many therapeutic uses, the preferred route/mode of administration is parenteral administration (e.g., intravenous or bolus injection, subcutaneous injection, intraperitoneal injection, and intramuscular injection). It will be appreciated by technicians that the route and/or mode of administration will vary depending upon the intended purpose. In some embodiments, the antibody or the antigen-binding fragment thereof or the pharmaceutical composition of the present invention is administered by intravenous injection or bolus injection.

### Detection uses

In a thirteenth aspect, the present application also provides a conjugate which comprises the antibody or the antigen-binding fragment thereof as described in the first aspect, the second aspect or the third aspect and a detectable label linked to the antibody or the antigen-binding fragment thereof.

In some embodiments, the detectable label is selected from an enzyme (e.g., horseradish peroxidase or alkaline phosphatase), a chemiluminescent reagent (e.g., acridinium ester compounds, luminol and derivatives thereof, or ruthenium derivatives), a fluorescent dye (e.g., fluorescein or fluorescent protein), a radionuclide, or a biotin.

In a fourteenth aspect, the present application also provides a kit which comprises the antibody or the antigen-binding fragment thereof as described in the first aspect, the second aspect or the third aspect, or the conjugate as described in the thirteenth aspect.

In some embodiments, the kit comprises a buffer for detection.

In some embodiments, the kit comprises the conjugate as described in the thirteenth aspect.

In some embodiments, the kit comprises the antibody or the antigen-binding fragment thereof as described in the first aspect, the second aspect or the third aspect, and a second antibody that specifically recognizes the antibody or the antigen-binding fragment thereof. Optionally, the second antibody further comprises a detectable label, such as an enzyme (e.g., horseradish peroxidase or alkaline phosphatase), a chemiluminescent reagent (e.g., acridinium ester compounds, luminol and derivatives thereof, or ruthenium derivatives), a fluorescent dye (e.g., fluorescein or fluorescent protein), a radionuclide, or a biotin.

In a fifteenth aspect, the present application also provides a method for detecting the presence or level of TSHR in a sample, which comprises using the antibody or the antigen-binding fragment thereof as described in the first aspect, the second aspect or the third aspect or the conjugate as described in the thirteenth aspect.

In some embodiments, the method is used for therapeutic purposes, diagnostic purposes, or non-therapeutic and non-diagnostic purposes.

In some embodiments, the method is an immunological assay, such as an immunoblot, an enzyme immunoassay (e.g., ELISA), a chemiluminescent immunoassay, a fluorescent immunoassay, or a radioimmunoassay.

In some embodiments, the method comprises using the conjugate as described in the thirteenth aspect.

In some embodiments, the method comprises using the antibody or the antigen-binding fragment thereof as described in the first aspect, the second aspect or the third aspect, and the method also comprises using a second antibody carrying a detectable label (e.g., an enzyme (e.g., horseradish peroxidase or alkaline phosphatase), a chemiluminescent reagent (e.g., acridinium ester compounds, luminol and derivatives thereof, or ruthenium derivatives), a fluorescent dye (e.g., fluorescein or fluorescent protein), a radionuclide, or a biotin) to detect the antibody or the antigen-binding fragment thereof.

In some embodiments, the method comprises: (1) contacting the sample with the antibody or the antigen-binding fragment thereof or the conjugate of the present invention; and (2) detecting the formation of an antigen-antibody immune complex, or detecting the amount of the immune complex. The formation of the immune complex indicates the presence of the TSHR or cells expressing the TSHR.

The present application also provides a method for diagnosing a TSHR-related disease, which comprises detecting the presence or level of the TSHR in a sample from a subject using the method described in the fifteenth aspect. In some embodiments, the presence of the TSHR or an increased level of the TSHR compared to a reference level (e.g., compared to a healthy control) indicates that the subject suffers from a TSHR-related disease.

In some embodiments, the TSHR-related disease is an autoimmune thyroid disease (e.g., Graves' disease or Graves' ophthalmopathy), thyroid cancer, or a combination thereof.

In a sixteenth aspect, the present application also provides the use of the antibody or the antigen-binding fragment thereof as described in the first aspect, the second aspect or the third aspect or the conjugate as described in the thirteenth aspect in the preparation of a detection reagent. The detection reagent is used for detecting the presence or level of the TSHR in a sample and/or diagnosing a TSHR-related disease.

In some embodiments, the detection reagent is used to detect the presence or level of the TSHR in a sample by the method as described in the fifteenth aspect.

In some embodiments, the detection reagent is used to detect the presence or level of the TSHR in a sample by the method as described in the fifteenth aspect to diagnose a TSHR-related disease. In some embodiments, the presence of the TSHR or an increased level of the TSHR compared to a reference level (e.g., compared to a healthy control) indicates that the subject suffers from a TSHR-related disease.

In some embodiments, the TSHR-related disease is an autoimmune thyroid disease (e.g., Graves' disease or Graves' ophthalmopathy), thyroid cancer, or a combination thereof.

In some embodiments, the sample is a tissue sample from a subject (e.g., a mammal, preferably a human, urine, or monkey).

### Definitions

In the present invention, unless otherwise specified, the scientific and technical terms used herein have the meanings commonly understood by those skilled in the art. Furthermore, the virology, biochemistry, and immunology laboratory operation procedures used herein are all conventional procedures widely used in the corresponding fields. Moreover, in order to better understand the present invention, definitions and explanations of relevant terms are provided below.

When the terms "for example", "e.g.", "such as", "including", "comprising", or variations thereof are used herein, these terms will not be considered as restrictive terms, but will be interpreted to mean "but not limited to" or "not limited to".

The terms "a", "an", "the", and similar referents in the context of describing the present invention (especially in the context of the following claims) should be construed to cover both the singular and the plural, unless otherwise indicated herein or clearly contradicted by context.

The term "antibody" as used herein refers to an immunoglobulin-derived molecule capable of specifically binding to a target antigen, and the immunoglobulin-derived molecule binds to the target antigen through at least one antigen-binding site located in a variable region thereof. When referring to the term "antibody", unless otherwise specified in the context, it includes not only an intact antibody but also an antigen-binding fragment capable of specifically binding to a target antigen. An "intact antibody" typically consists of two pairs of polypeptide chains, each pair having one light chain (LC) and one heavy chain (HC). Light chains of the antibody can be classified as kappa (κ) and lambda (λ) light chains. Heavy chains can be classified as µ, δ, γ, α or ε, and the isotype of the antibody is respectively defined as IgM, IgD, IgG, IgA, and IgE. Within light and heavy chains, the variable and constant regions are joined by a "J" region of about 12 or more amino acids, and the heavy chain also comprises a "D" region of about 3 or more amino acids. Each heavy chain consists of a heavy chain variable region (VH) and a heavy chain constant region (CH). The heavy chain constant region consists of three domains (CH1, CH2, and CH3). Each light chain consists of a light chain variable region (VL) and a light chain constant region (CL). The light chain constant region consists of one domain CL. A constant domain is not directly involved in binding of an antibody to an antigen, but exhibits various effector functions, such as mediating the binding of immunoglobulins to host tissues or factors, including various cells of an immune system (e.g., effector cells) and a first component (C1q) of a classical complement system. VH and VL regions can be further subdivided into regions with high variability (called complementarity determining regions (CDRs)) interspersed with relatively conserved regions (called framework regions (FRs)). Each of the VH and VL consists of three CDRs and four FRs arranged from an amino terminal to a carboxyl terminal in the following order: FR1, CDR1, FR2, CDR2, FR3, CDR3, and FR4. The variable regions (VH and VL) of each heavy chain/light chain pair respectively form an antigen-binding site. The distribution of amino acids in each region or domain can be based on the definitions in Kabat, Sequences of Proteins of Immunological Interest (National Institutes of Health, Bethesda, Md. (1987 and 1991)), or Chothia & Lesk (1987) J. Mol. Biol. 196:901-917; Chothia et al. (1989) Nature 342:878-883.

As used herein, the term "complementarity determining region" or "CDR" refers to amino acid residues responsible for antigen binding in a variable region of an antibody. Each of the variable regions of the heavy and light chains comprises three CDRs, named CDR1, CDR2, and CDR3. The precise boundaries of these CDRs can be defined according to various numbering systems known in the art, such as the definitions in the Kabat numbering system (Kabat et al., Sequences of Proteins of Immunological Interest, 5th Ed. Public Health Service, National Institutes of Health, Bethesda, Md., 1991), the Chothia numbering system (Chothia & Lesk (1987) J. Mol. Biol. 196:901-917; Chothia et al. (1989) Nature 342:878-883), or the IMGT numbering system (Lefranc et al., Dev. Comparat. Immunol. 27:55-77, 2003). For a given antibody, those skilled in the art will readily identify the CDRs defined by each numbering system. Furthermore, the corresponding relationship between different numbering systems is well known to those skilled in the art (for example, see Lefranc et al., Dev. Comparat. Immunol. 27:55-77, 2003).

In the present invention, the CDR comprised in the antibody or the antigen-binding fragment thereof of the present invention can be determined according to various numbering systems known in the art. In some embodiments, the CDR comprised in the antibody or the antigen-binding fragment thereof of the present invention is preferably determined by the Kabat, Chothia or IMGT numbering system.

As used herein, the term "framework region" or "FR" residues refer to those amino acid residues in variable regions of an antibody other than the CDR residues as defined above.

The term "antibody" is not limited to any particular method of producing the antibody. For example, antibodies include recombinant antibodies, monoclonal antibodies, and polyclonal antibodies. Antibodies can be of different isotypes, for example, IgG (e.g., IgG1, IgG2, IgG3, or IgG4 subtype), IgA1, IgA2, IgD, IgE, or IgM antibodies.

As used herein, the term "antigen-binding fragment" of an antibody refers to a polypeptide comprising a fragment of a full-length antibody that retains the ability to specifically bind to the same antigen that the full-length antibody binds to and/or competes with the full-length antibody for specific binding to the antigen, which is also referred to as an "antigen-binding portion". Generally, see Fundamental Immunology, Ch. 7 (Paul, W., ed., 2nd ed., Raven Press, N.Y. (1989), which is incorporated herein by reference in its entirety for all purposes. The antigen-binding fragment of an antibody can be produced by recombinant DNA technologies or by enzymatic or chemical cleavage of an intact antibody. Non-limiting examples of the antigen-binding fragment include Fab, Fab', F(ab')₂, Fd, Fv, disulfide-linked Fv, scFv, di-scFv, a diabody, a single domain antibody, and a polypeptide comprising at least a portion of an antibody sufficient to confer a specific antigen-binding ability to the polypeptide. Engineered antibody variants are reviewed in Holliger et al., 2005; Nat Biotechnol, 23: 1126-1136.

As used herein, the term "Fd" means an antibody fragment consisting of VH and CH1 domains; the term "Fab fragment" means an antibody fragment consisting of VL, VH, CL, and CH1 domains; the term "F(ab')₂ fragment" means an antibody fragment comprising two Fab fragments linked by a disulfide bridge on a hinge region; and the term "Fab' fragment" means a fragment obtained after reducing disulfide bonds linking two heavy chain fragments in the F(ab')₂ fragment, consisting of a complete light chain and the Fd fragment (consisting of VH and CH1 domains) of a heavy chain.

As used herein, the term "Fv" means an antibody fragment consisting of the VL and VH domains of a single arm of an antibody. The Fv fragment is generally considered to be the smallest antibody fragment that capable of forming a complete antigen-binding site. It is generally believed that six CDRs confer an antigen-binding specificity to an antibody. However, a single variable region (e.g., the Fd fragment, which comprises only three CDRs specific for an antigen) also has the ability to recognize and bind to an antigen, although the affinity thereof may be lower than that of a complete binding site.

As used herein, the term "Fc" means an antibody fragment formed by the second and third constant regions of a first heavy chain of an antibody binding to the second and third constant regions of a second heavy chain of the antibody through disulfide bonds. The Fc fragment of an antibody has a variety of different functions but is not involved in antigen binding.

As used herein, the term "scFv" refers to a single polypeptide chain comprising VL and VH domains, wherein the VL and VH are linked by a linker (see, e.g., Bird et al., Science 242:423-426 (1988); Huston et al., Proc. Natl. Acad. Sci. USA 85:5879-5883 (1988); and Pluckthun, The Pharmacology of Monoclonal Antibodies, Vol. 113, Roseburg and Moore, eds., Springer-Verlag, New York, pp. 269-315 (1994)). Such scFv molecules may have a general structure: NH₂-VL-Linker-VH-COOH or NH₂-VH-Linker-VL-COOH. A suitable linker in the prior art consists of repeated GGGGS amino acid sequences or variants thereof. For example, a linker having an amino acid sequence (GGGGS)₄ may be used, but a variant thereof may also be used (Holliger et al. (1993), Proc. Natl. Acad. Sci. USA 90: 6444-6448). Other linkers that can be used in the present invention are described in Alfthan et al. (1995), Protein Eng. 8:725-731, Choi et al. (2001), Eur. J. Immunol. 31: 94-106, Hu et al. (1996), Cancer Res. 56:3055-3061, Kipriyanov et al. (1999), J. Mol. Biol. 293:41-56, and Roovers et al. (2001), Cancer Immunol. In some cases, a disulfide bond may also exist between the VH and VL of the scFv. In some embodiments of the present invention, the scFv may form a di-scFv, which refers to two or more single scFvs in series to form an antibody. In some embodiments of the present invention, the scFv may form a (scFv)₂, which refers to two or more single scFvs in parallel to form an antibody.

As used herein, the term "diabody" means that the VH and VL domains thereof are expressed on a single polypeptide chain, but using a linker that is too short to allow pairing between two domains of the same chain, thereby forcing the domains to pair with the complementary domains of another chain and form two antigen-binding sites (see, e.g., Holliger P. et al., Proc. Natl. Acad. Sci. USA 90:6444-6448 (1993), and Poljak R.J. et al., Structure 2:1121-1123 (1994)).

As used herein, the term "single domain antibody (sdAb)" has the meaning commonly understood by those skilled in the art, and refers to an antibody fragment consisting of a single monomeric variable antibody domain (e.g., single heavy chain variable region), which retains the ability to specifically bind to the same antigen as a full-length antibody. A single domain antibody is also called a nanobody.

As used herein, the term "bispecific antibody" refers to an antibody having a binding specificity for two different antigens (or epitopes). The term "multispecific antibody" refers to an antibody having a binding specificity for at least more than two (e.g., three or four) different antigens (or epitopes). The bispecific antibody or the multispecific antibody comprises multiple antigen-binding domains having a binding specificity for different antigens (or epitopes), and thus is able to bind to at least two different binding sites and/or target molecules. Each antigen-binding domain comprised in the bispecific antibody or the multispecific antibody can be independently selected from a full-length antibody (e.g., IgG antibody) or an antigen-binding fragment thereof (e.g., Fv fragment, Fab fragment, F(ab')₂ fragment, or scFv). In some cases, individual antigen-binding domains are linked by peptide linkers.

Each of the above antibody fragments retains the ability to specifically bind to the same antigen as the full-length antibody, and/or competes with the full-length antibody for specific binding to the antigen.

An antigen-binding fragment of an antibody (e.g., the above-mentioned antibody fragment) can be obtained from a given antibody (e.g., antibody provided in the present invention) by a conventional technology known to those skilled in the art (e.g., recombinant DNA technology or enzymatic or chemical cleavage method), and the antigen-binding fragment of the antibody can be screened for specificity in the same manner as for an intact antibody.

As used herein, the term "humanized antibody" refers to a non-human antibody that is genetically engineered, and the amino acid sequence thereof is modified to increase the homology with the sequence of a human antibody. Generally speaking, all or part of CDR regions of a humanized antibody are derived from a non-human antibody (donor antibody), and all or part of non-CDR regions (e.g., variable region FR and/or constant region) are derived from a human immunoglobulin (receptor antibody). In some embodiments, CDR regions of a humanized antibody are derived from a non-human antibody (donor antibody), and all or part of non-CDR regions (e.g., variable region FR and/or constant region) are derived from a human immunoglobulin (receptor antibody). A humanized antibody generally retains the desired properties of a donor antibody, including but not limited to, antigen specificity, affinity, reactivity, etc. In the present application, the donor antibody may be a murine antibody having desired properties (e.g., antigen specificity, affinity, reactivity, etc.). To prepare a humanized antibody, CDR regions of a donor antibody can be inserted into human framework sequences by a method known in the art. In some cases, the human framework sequence may comprise amino acid mutations that are substituted by corresponding non-human residues. In addition, a humanized antibody may also comprise residues that are not found in a variable region (e.g., light chain variable region or heavy chain variable region) of an initial donor antibody or in a human framework sequence to further improve or optimize the performance of the humanized antibody.

As used herein, the term "chimeric antibody" refers to an antibody in which a portion of the light chain and/or the heavy chain thereof is derived from one antibody (which may be derived from a particular species or belong to a particular antibody type or subtype), and another portion of the light chain and/or the heavy chain is derived from another antibody (which may be derived from the same or different species or belong to the same or different antibody types or subtypes), but in any case, the binding activity to a target antigen is still retained (U.S.P 4,816,567 to Cabilly et al.; Morrison et al., Proc. Natl. Acad. Sci. USA, 81:6851-6855 (1984)). In some embodiments, the term "chimeric antibody" may include an antibody in which the heavy chain variable region and light chain variable region of the antibody are derived from a first antibody, and the heavy chain constant region and light chain constant region of the antibody are derived from a second antibody.

As used herein, the term "identity" is used for referring to the matching situation of sequences between two polypeptides or between two nucleic acids. To determine the percent identity between two amino acid sequences or two nucleic acid sequences, the sequences are aligned for optimal comparison purposes (e.g., gaps can be introduced in a first amino acid or nucleic acid sequence for optimal alignment with a second amino acid or nucleic acid sequence). Then, amino acid residues or nucleotides at corresponding amino acid positions or nucleotide positions are compared. When a position in the first sequence is occupied by the same amino acid residue or nucleotide as the corresponding position in the second sequence, molecules are identical at that position. The percent identity between two sequences is a function of the number of identical positions shared by the sequences (i.e., percent identity = number of identical overlapping positions/total number of positions x 100%). In some embodiments, two sequences have the same length.

The determination of the percent identity between two sequences can also be achieved by a mathematical algorithm. A non-limiting example of a mathematical algorithm for comparison of two sequences is improved in algorithms of Karlin and Altschul, 1990, Proc. Natl. Acad. Sci. U.S.A. 87:2264-2268, as in Karlin and Altschul, 1993, Proc. Natl. Acad. Sci. U.S.A. 90:5873-5877. Such an algorithm is integrated into NBLAST and XBLAST programs of Altschul et al., 1990, J. Mol.Biol. 215:403.

As used herein, the term "variant", in the context of polypeptides (including polypeptides), also refers to a polypeptide or peptide comprising an amino acid sequence that has been altered by the introduction of amino acid residue substitutions, deletions or additions. In some cases, the term "variant" also refers to a polypeptide or peptide that has been modified (i.e., by covalent linkage of any type of molecule to the polypeptide or peptide). For example, but not limitation, the polypeptide can be modified, for example, by glycosylation, acetylation, pegylation, phosphorylation, amidation, derivatization by known protecting/blocking groups, proteolytic cleavage, linkage to cellular ligands or other proteins, etc. Derived polypeptides or peptides can be produced by chemical modification using technologies known to those skilled in the art. The technologies include but not limited to specific chemical cleavage, acetylation, formylation, metabolic synthesis of tunicamycin, etc. Furthermore, the variant has similar, identical or improved functions as the polypeptide or peptide from which it is derived.

As used herein, the term "specific binding" refers to a non-random binding reaction between two molecules, such as the reaction between an antibody and a targeted antigen. The strength or affinity of a specific binding interaction can be expressed in terms of an equilibrium dissociation constant (K_{D}) for the interaction. In the present invention, the term "K_{D}" refers to a dissociation equilibrium constant for a specific antibody-antigen interaction, which is used for describing the binding affinity between an antibody and an antigen. The smaller the equilibrium dissociation constant, the tighter the antibody-antigen binding, and the higher the affinity between the antibody and the antigen.

The specific binding properties between two molecules can be determined by a method known in the art. One method involves measurement of the rates of formation and dissociation of antigen-binding sites/antigen complexes. Both the "association rate constant" (ka or kon) and the "dissociation rate constant" (kdis or koff) can be calculated by the concentration and the actual rates of association and dissociation (see Malmqvist M, Nature,1993, 361:186-187). The ratio of kdis/kon is equal to the dissociation constant K_{D} (see Davies et al., Annual Rev Biochem, 1990; 59:439-473). The values of the K_{D}, kon and kdis can be measured by any effective method. In some embodiments, the dissociation constant can be measured in Biacore by surface plasmon resonance (SPR). In addition, bioluminescence interferometry or Kinexa can be used for measuring the dissociation constant.

As used herein, the detectable label of the present invention may be any substance detectable by fluorescent, spectroscopic, photochemical, biochemical, immunological, electrical, optical or chemical means. Such labels are well known in the art, and examples thereof include, but are not limited to, enzymes (e.g., horseradish peroxidase, alkaline phosphatase, β-galactosidase, urease, glucose oxidase, etc.), radionuclides (e.g., ³H, ¹²⁵I, ³⁵S, ¹⁴C, or ³²P), fluorescent dyes (e.g., fluorescein isothiocyanate (FITC), fluoresceins, tetramethylrhodamine isothiocyanate (TRITC), fluorescent proteins (e.g., phycoerythrin (PE)), Texas Red, rhodamine, quantum dots or cyanine dye derivatives (e.g., Cy7 and Alexa 750)), luminescent substances (e.g., chemiluminescent reagents, such as acridinium ester compounds, luminol and derivatives thereof, and ruthenium derivatives such as terpyridine ruthenium), magnetic beads (e.g., Dynabeads^{®}), calorimetric labels such as colloidal gold or colored glass or plastic (e.g., polystyrene, polypropylene, latex, etc.) beads, and biotins for binding to avidins (e.g., streptavidin) modified with the above labels. In some embodiments, the detectable label is selected from an enzyme (e.g., horseradish peroxidase or alkaline phosphatase), a chemiluminescent reagent (e.g., acridinium ester compounds, luminol and derivatives thereof, or ruthenium derivatives), a fluorescent dye (e.g., fluorescein or fluorescent protein), a radionuclide, or a biotin. In some embodiments, the detectable label described above can be linked to the antibody or the antigen-binding fragment thereof of the present invention through linkers of different lengths to reduce potential steric hindrance.

As used herein, the term "vector" refers to a nucleic acid delivery vehicle into which a polynucleotide can be inserted. When the vector can express the protein encoded by the inserted polynucleotide, the vector is called an expression vector. The vector can be introduced into the host cell by transformation, transduction or transfection, so that the genetic material elements carried by the vector are expressed in the host cell. Vectors are well known to those skilled in the art, and include but are not limited to: plasmids; phagemids; cosmids; artificial chromosomes, such as yeast artificial chromosomes (YACs), bacterial artificial chromosomes (BACs), or P1-derived artificial chromosomes (PACs); and phages such as λ phages or M13 phages and animal viruses. Animal viruses that can be used as vectors include, but are not limited to, retroviruses (including lentiviruses), adenoviruses, adeno-associated viruses, herpes viruses (e.g., herpes simplex viruses), poxviruses, baculoviruses, papillomaviruses, and papovaviruses (e.g., SV40). A vector may comprise a variety of elements for controlling expression, including, but not limited to, promoter sequences, transcription initiation sequences, enhancer sequences, selection elements, and reporter genes. In addition, the vector may also comprise a replication origin.

As used herein, the term "host cell" refers to a cell that can be used for introducing a vector, including but not limited to prokaryotic cells such as Escherichia coli or Bacillus subtilis, fungal cells such as yeast cells or Aspergillus, insect cells such as S2 Drosophila cells or Sf9, or animal cells such as fibroblasts, CHO cells, COS cells, NSO cells, HeLa cells, BHK cells, HEK 293 cells or human cells.

As used herein, the term "conservative substitution" means an amino acid substitution that does not adversely affect or alter the desired properties of the protein/polypeptide comprising the amino acid sequence. For example, conservative substitutions can be introduced by standard technologies known in the art, such as site-directed mutagenesis and PCR-mediated mutagenesis. Conservative amino acid substitutions include replacement of an amino acid residue with an amino acid residue having a similar side chain, such as a residue that is physically or functionally similar to the corresponding amino acid residue (e.g., having similar size, shape, charge and chemical properties, including the ability to form covalent bonds or hydrogen bonds, etc.). Families of amino acid residues having similar side chains have been defined in the art. These families include amino acids with basic side chains (e.g., lysine, arginine, and histidine), acidic side chains (e.g., aspartic acid and glutamic acid), uncharged polar side chains (e.g., glycine, asparagine, glutamine, serine, threonine, tyrosine, cysteine, and tryptophan), nonpolar side chains (e.g., alanine, valine, leucine, isoleucine, proline, phenylalanine, and methionine), β-branched side chains (e.g., threonine, valine, and isoleucine), and aromatic side chains (e.g., tyrosine, phenylalanine, tryptophan, and histidine). Thus, it is preferred to replace a corresponding amino acid residue with another amino acid residue from the same side chain family. Methods for identifying conservative amino acid substitutions are well known in the art (see, e.g., Brummell et al., Biochem. 32:1180-1187 (1993); Kobayashi et al. Protein Eng. 12(10):879-884 (1999); and Burks et al. Proc. Natl Acad. Set USA 94:412-417 (1997), which is incorporated herein by reference).

Twenty conventional amino acids mentioned herein are written in accordance with conventional usage. See, e.g., Immunology-A Synthesis (2nd Edition, E. S. Golub and D. R. Gren, Eds., Sinauer Associates, Sunderland, Mass. (1991)), which is incorporated herein by reference. In the present invention, the terms "polypeptide" and "protein" have the same meaning and are used interchangeably. Moreover, in the present invention, amino acids are generally represented by single-letter and three-letter abbreviations well known in the art. For example, alanine can be represented by A or Ala.

As used herein, the term "pharmaceutically acceptable carrier and/or excipient" refers to a carrier and/or excipient that is pharmacologically and/or physiologically compatible with a subject and active ingredients, which is well known in the art (see, e.g., Remington's Pharmaceutical Sciences. Edited by Gennaro AR, 19th ed. Pennsylvania: Mack Publishing Company, 1995), and includes, but is not limited to: pH adjusting agents, surfactants, adjuvants, ionic strength enhancers, diluents, reagents for maintaining osmotic pressure, reagents for delaying absorption, preservatives, and stabilizers. For example, pH adjusting agents include, but are not limited to, phosphate buffers. Surfactants include, but are not limited to, cationic, anionic or nonionic surfactants, such as Tween-80. Ionic strength enhancers include, but are not limited to, sodium chloride. Reagents for maintaining osmotic pressure include, but are not limited to, sugars, NaCl, and analogs thereof. Reagents for delaying absorption include, but are not limited to, monostearates and gelatin. Diluents include, but are not limited to, water, aqueous buffers (e.g., buffered saline), alcohols, polyols (e.g., glycerol), etc. Preservatives include, but are not limited to, various antibacterial reagents and antifungal reagents such as thimerosal, 2-phenoxyethanol, parabens, chlorobutanol, phenol, sorbic acid, etc. Stabilizers have the meanings generally understood by those skilled in the art, which are capable of stabilizing the desired activity of active ingredients in drugs, and include, but are not limited to, sodium glutamate, gelatin, SPGA, sugars (e.g., sorbitol, mannitol, starch, sucrose, lactose, dextran, or glucose), amino acids (e.g., glutamic acid and glycine), proteins (e.g., dried whey, albumin or casein), or degradation products thereof (e.g., lactalbumin hydrolysate), etc. In some exemplary embodiments, the pharmaceutically acceptable carrier or excipient comprises sterile injectable liquid (e.g., an aqueous or non-aqueous suspension or solution). In some exemplary embodiments, such sterile injectable liquid is selected from water for injection (WFI), bacteriostatic water for injection (BWFI), a sodium chloride solution (e.g., 0.9% NaCl), a glucose solution (e.g., 5% glucose), a solution comprising a surfactant (e.g., 0.01% polysorbate 20), a pH buffer (e.g., phosphate buffer), a Ringer's solution, and any combination thereof.

As used herein, the term "prevention" refers to a method implemented for preventing or delaying the occurrence of a disease or disorder or symptom in a subject. As used herein, the term "treatment" refers to a method implemented for obtaining beneficial or desired clinical results. For purposes of the present invention, beneficial or desired clinical results include, but are not limited to, alleviation of symptoms, reduction in the extent of diseases, stabilization (i.e., no longer worsening) of disease states, delay or slowing of disease progression, amelioration or palliation of disease states, and relief of symptoms (regardless partial or complete), regardless detectable or undetectable. In addition, the "treatment" may also mean prolonging the survival time as compared to the expected survival time (e.g., survival time without treatment).

As used herein, the term "subject" refers to a mammal, such as a human, murine, or monkey. In some embodiments, the subject (e.g., human, murine, or monkey) suffers from a TSHR-related disease (e.g., Graves' disease or Graves' ophthalmopathy), or has the risk of suffering from the above disease.

As used herein, the term "effective amount" refers to an amount sufficient to achieve or at least partially achieve desired effects. For example, an effective amount for preventing a disease (e.g., Graves' disease or Graves' ophthalmopathy) refers to an amount sufficient to prevent, inhibit or delay the occurrence of the disease; and an effective amount for treating a disease refers to an amount sufficient to cure or at least partially inhibit the disease and complications thereof in a patient suffering from the disease. Determination of such effective amounts is completely within the capability of those skilled in the art. For example, amounts effective for therapeutic use will depend on the severity of the disease to be treated, the overall state of the immune system of the patient, the general conditions of the patient such as age, weight and sex, the manner of administration of a drug, and other therapies administered simultaneously.

### Beneficial effects of the present invention

The antibody provided in the present application has high affinity to human TSHR and has cross-binding activity against human/murine/monkey TSHR. In addition, the antibody of the present application has a significant inhibitory effect on the activity of TSHR, and can inhibit the downstream signal pathway mediated by the binding of the TSHR with an activating antibody by blocking the binding of the TSHR with the activating antibody. After verification, the antibody of the present application has the function of significantly inhibiting the secretion of hyaluronic acid, interleukin-6 and interleukin-8 in primary orbital fibroblasts of GO patients in in-vitro functional experiments.

The embodiments of the present invention will be described in detail below in conjunction with the accompanying drawings and examples, but those skilled in the art will understand that the following accompanying drawings and examples are only used for illustrating the present invention rather than limiting the scope of the present invention. Various purposes and advantages of the present invention will become apparent to those skilled in the art according to the accompanying drawings and the following detailed descriptions of the preferred embodiments.

### BRIEF DESCRIPTION OF THE DRAWINGS

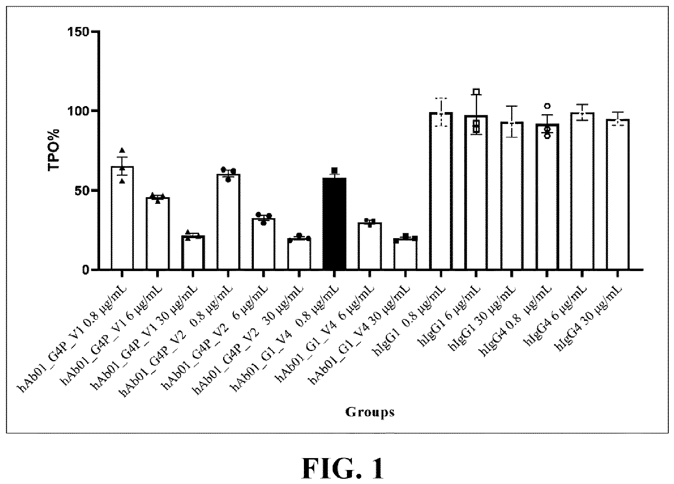

FIG. 1: Engineered antibodies inhibit production of TPO in thyroid cells induced by serum of GD patients.

### Sequence information

The description of the sequences involved in the present application is provided in the following table.

**Table 1: Sequence information**

| SEQ ID NO: | Sequence description |
|---|---|
| 1 | VH amino acid sequence of murine antibody mAb001 |
| 2 | VH CDR1 amino acid sequence of murine antibody mAb001 |
| 3 | VH CDR2 amino acid sequence of murine antibody mAb001 |
| 4 | VH CDR3 amino acid sequence of murine antibody mAb001 |
| 5 | VL amino acid sequence of murine antibody mAb001 |
| 6 | VL CDR1 amino acid sequence of murine antibody mAb001 |
| 7 | VL CDR2 amino acid sequence of murine antibody mAb001 |
| 8 | VL CDR3 amino acid sequence of murine antibody mAb001 |
| 9 | VH amino acid sequence of murine antibody mAb012 |
| 10 | VH CDR1 amino acid sequence of murine antibody mAb012 |
| 11 | VH CDR2 amino acid sequence of murine antibody mAb012 |
| 12 | VH CDR3 amino acid sequence of murine antibody mAb012 |
| 13 | VL amino acid sequence of murine antibody mAb012 |
| 14 | VL CDR1 amino acid sequence of murine antibody mAb012 |
| 15 | VL CDR2 amino acid sequence of murine antibody mAb012 |
| 16 | VL CDR3 amino acid sequence of murine antibody mAb012 |
| 17 | VH amino acid sequence of murine antibody mAb021 |
| 18 | VH CDR1 amino acid sequence of murine antibody mAb021 |
| 19 | VH CDR2 amino acid sequence of murine antibody mAb021 |
| 20 | VH CDR3 amino acid sequence of murine antibody mAb021 |
| 21 | VL amino acid sequence of murine antibody mAb021 |
| 22 | VL CDR1 amino acid sequence of murine antibody mAb021 |
| 23 | VL CDR2 amino acid sequence of murine antibody mAb021 |
| 24 | VL CDR3 amino acid sequence of murine antibody mAb021 |
| 25 | Amino acid sequence of human IgG4-S228P heavy chain constant region |
| 26 | Amino acid sequence of human kappa light chain constant region |
| 27 | VH amino acid sequence of humanized antibody cAb01-VH1-DA-VL2/cAb01-VH1-DA-VL3 |
| 28 | VL amino acid sequence of humanized antibody cAb01-VH1-DA-VL2/cAb01-VH2-DA-VL2/cAb01-VH3-DA-VL2 |
| 29 | VH amino acid sequence of humanized antibody cAb01-VH2-DA-VL2/cAb01-VH2-DA-VL3 |
| 30 | VH amino acid sequence of humanized antibody cAb01-VH3-DA-VL2 |
| 31 | VL amino acid sequence of humanized antibody cAb01-VH1-DA-VL3/cAb01-VH2-DA-VL3 |
| 32 | VH amino acid sequence of humanized antibody cAb12-VH1-QG&S-VL3 |
| 33 | VL amino acid sequence of humanized antibody cAb12-VH1-QG&S-VL3/cAb12-VH3-QG&S-VL3 |
| 34 | VH amino acid sequence of humanized antibody cAb12-VH3-QG&S-VL3 |
| 35 | VH amino acid sequence of humanized antibody cAb21-VH2-VL2 |
| 36 | VL amino acid sequence of humanized antibody cAb21-VH2-VL2 |
| 37 | VH amino acid sequence of humanized antibody cAb21-VH1-VL3 |
| 38 | VL amino acid sequence of humanized antibody cAb21-VH1-VL3 |
| 39 | VH CDR2 amino acid sequence of humanized antibody cAb01-VH1-DA-VL2/cAb01-VH2-DA-VL2/cAb01-VH3-DA-VL2 |
| 40 | VH CDR2 amino acid sequence of humanized antibody cAbl2-VH1-QG&S-VL3/cAb 12- VH3-QG&S- VL3 |
| 41 | VH CDR3 amino acid sequence of humanized antibody cAb12-VH1-QG&S-VL3/cAb12-VH3-QG&S-VL3 |
| 42 | Amino acid sequence of heavy chain constant region of human IgG4-S228P/M252Y/S254T/T256E |
| 43 | Amino acid sequence of heavy chain constant region of human IgG4-S228P/L235E/M252Y/S254T/T256E |
| 44 | Amino acid sequence of heavy chain constant region of human IgG1 |
| 45 | Amino acid sequence of heavy chain constant region of human IgG1-L234A/L235A/M252Y/S254T/T256E |
| 46 | Amino acid sequence of heavy chain of hAb01 G4P |
| 47 | Amino acid sequence of heavy chain of hAb01_G4P_V1 |
| 48 | Amino acid sequence of heavy chain of hAb01 G4P V2 |
| 49 | Amino acid sequence of heavy chain of hAb01 G1 |
| 50 | Amino acid sequence of heavy chain of hAb01_G1_V4 |
| 51 | Amino acid sequence of light chain of hAb01_G4P/hAb01_G4P V1/hAb01 G4P V2/hAb01_G1/hAb01_G1_V4 |

### DETAILED DESCRIPTION

The present invention will be described with reference to the following examples which are intended to illustrate the present invention rather than to limit the present invention.

Unless otherwise specified, the molecular biology experimental methods and immunoassays used in the present invention are basically performed based on the methods described in J. Sambrook et al., Molecular Cloning: Laboratory Manual, 2nd edition, Cold Spring Harbor Laboratory Press, 1989, and F. M. Ausubel et al., Compiled Molecular Biology Experiment Guide, 3rd edition, John Wiley & Sons, Inc., 1995. Restriction endonucleases were used according to the conditions recommended by product manufacturers. Those skilled in the art will appreciate that the examples describe the present invention by way of examples and are not intended to limit the scope of protection claimed for the present invention.

### Example 1: Antibody preparation

### 1. Preparation of hybridoma antibodies:

1.1 After immunizing mice with human TSHR antigens, mice with higher titers were selected for cell fusion and hybridoma preparation.

1.2 Myeloma cells were prepared; a counter was used for counting and detecting viability before fusion; SP2/0 cells were collected; centrifugation was performed at 400 g for 5 min at a room temperature; the culture solution was discarded, and the cell pellet was resuspended with 20 ml of fusion culture solution.

1.3 Spleen cells were collected and cultured, mice with relatively strong immunogen response were selected for preparation of hybridoma cells; mice were euthanized with carbon dioxide according to the method approved by IACUC; pre-fusion plasma was collected, and serum (FB) was separated and collected as a positive control for hybridoma supernatant screening.

1.4 The mice were soaked in 70% alcohol for disinfection and then immediately transferred to a biosafety cabinet for operation; the spleen was collected and placed in a sterile culture dish; the spleen was placed on a filter screen, and was repeatedly squeezed with a stopper of a sterile syringe until there were no obvious clumps; and then, the filter screen was rinsed with the fusion culture solution to obtain a spleen cell suspension which is then completely transferred to a 50 ml centrifuge tube.

1.5 Centrifugation was performed at 400 g for 5 min, the supernatant was discarded; fusion cells were prepared by electrofusion; the spleen cell pellet was resuspended with 5 ml of red blood cell lysis buffer and then allowed to stand at 4°C for 5 min, and the reaction was terminated with 50 ml of DEME culture solution added with 10% FBS. The spleen cell pellet was collected by centrifugation, then the fusion culture solution was added to resuspend the cells, and the centrifuge tube was inverted 3-5 times and then counted. Centrifugation was performed at 400 g for 5 min, the supernatant was discarded, the cell pellet was retained, and then, the cells were resuspended with 40 ml of fusion culture solution. The spleen cells were allowed to stand at a room temperature for 2-3 min and then transferred to a new 50 ml centrifuge tube. SP2/0 cells were added to the spleen cells at a ratio of 4:1 between spleen cells and SP2/0 cells, and the volume was fixed to 50 ml with the fusion culture solution. The cell mixed solution was centrifuged, the supernatant was discarded, and the cell pellet was loosened by repeatedly tapping the bottom of the tube. Fusion: For electrofusion, the cell mixture was placed in an electrode tank, and electroporation fusion was performed according to an optimized program.

1.6 After electrofusion, the fusion cells were allowed to stand in the electrode tank for additional 10 min. Cellls were resuspended with a DMEM culture solution added with 20% FBS and HT, and seeded to a 96-well microplate at a volume of 100 µl per well. After 24 h, the DMEM culture solution added with 20% FBS and HAT was added to the well plate, 100 µl per well. All fusion plates were transferred back to a CO₂ incubator with 5% of CO₂ at 37°C. The cell growth rate and microbial contamination were monitored daily. When hybridoma clones grew to a size of 1-2 mm in diameter (generally 10-14 d after fusion), hybridoma supernatants were screened with Acumen.

1.7 Subcloning and amplification culture. Positive clones were transferred to a 24-well plate and cultured with an amplification culture solution. The hybridoma culture supernatant was collected for FACS detection. Positive mother clones were subcloned to obtain single clones by limiting dilution. A subclone 96-well plate was placed in the CO₂ incubator and cultured for 7 d, and then, the activity of the screened supernatant was detected. To ensure single clones, 1-2 rounds of subcloning were required. According to the screening results, four single clones with the best activity were selected from each subclone plate and transferred to a 24-well plate for amplification culture.

1.8 Cryopreservation of cells. Amplified subclone cell lines were cryopreserved and stored in a DMEM culture solution added with 20% FBS and 10% DMSO, and placed in a liquid nitrogen tank for long-term storage.

Murine monoclonal antibodies mAb001, mAb012 and mAb021 were prepared by the above method, and CDR and variable region sequences thereof were shown in Table 2.

**Table 2 Variable region and CDR sequence information of murine antibodies**

| Antibody | VH | | | VH | VL | | | VL |
|---|---|---|---|---|---|---|---|---|
| | CDR1 | CDR2 | CDR3 | | CDR1 | CDR2 | CDR3 | |
| mAb001 | SEQ ID NO: 2 | SEQ ID NO: 3 | SEQ ID NO: 4 | SEQ ID NO: 1 | SEQ ID NO: 6 | SEQ ID NO: 7 | SEQ ID NO: 8 | SEQ ID NO: 5 |
| mAb012 | SEQ ID NO: 10 | SEQ ID NO: 11 | SEQ ID NO: 12 | SEQ ID NO: 9 | SEQ ID NO: 14 | SEQ ID NO: 15 | SEQ ID NO: 16 | SEQ ID NO: 13 |
| mAb021 | SEQ ID NO: 18 | SEQ ID NO: 19 | SEQ ID NO: 20 | SEQ ID NO: 17 | SEQ ID NO: 22 | SEQ ID NO: 23 | SEQ ID NO: 24 | SEQ ID NO: 21 |

### 2. Humanization of murine antibodies:

Humanized antibodies were designed using CDR grafting. The sequence closest to the murine sequence in a BLAST human antibody was used as a variable region framework to replace a murine framework region. The CDRs of the parent antibody were grafted into a human receptor, and all sequences were analyzed for post-translational modifications (PTMs), such as isomerization, deamidation, and glycosylation risks. Appropriate PTMs were designed to remove mutations to ultimately obtain each humanized light chain and humanized heavy chain for each parent antibody. The humanized light and heavy chain variable regions of each parent antibody were paired with each other for affinity ranking experiments. Then, the heavy chain variable region and the light chain variable region were linked to the human IgG4-S228P heavy chain constant region (SEQ ID NO: 25) and the human kappa light chain constant region (SEQ ID NO: 26) respectively to obtain full-length sequences of the heavy and light chains of humanized antibodies.

DNA sequences encoding the heavy and light chains of humanized antibodies were synthesized and inserted into a pcDNA3.4 vector to construct a full-length antibody expression plasmid. Expression of humanized antibodies was performed in an Expi293F cell culture, and the supernatant was purified with a protein A affinity column. The purified antibody buffer was exchanged into PBS with a PD-10 desalting column. The concentration and purity of the purified protein were determined by OD280 and SDS-PAGE respectively.

Humanized antibodies of each murine monoclonal antibody were obtained by the above method, and the amino acid sequences of the light and heavy chain variable regions of each humanized antibody were set forth in SEQ ID NOs: 27-38.

### Example 2: murine antibody cell binding experiment

### Experimental operation steps:

The binding strength of murine antibodies to 293-human TSHR, 293-mouse TSHR, and 293-monkey TSHR overexpressing cell lines (the cells used were 293 cells overexpressing TSHR derived from human, monkey or mouse, wherein the amino acid sequences of the TSHR derived from the human, monkey and mouse were shown in P16473, A0A2K5X226, and P47750 Uniprot respectively) was detected by flow cytometry (FACS). The experimental method was as follows: cells in the logarithmic growth phase were collected and counted with a cell counter (Countstar; IC1000); the cells were resuspended in a cold FACS buffer which is prepared from 500 ml of PBS (HyClone, SH30256.01B) added with 50 ml of FBS (BI; 04-002-1A), and the cell density was adjusted to 5x10⁶ cells/ml; after being allowed to stand at a room temperature for 10 to 20 min, the cell suspension was added to an FACS plate (Corning, 3799), 100 µL per well, centrifugation was performed, and the supernatant was discarded; the antibody to be detected was diluted with an FACS buffer with the initial concentration of 200 nM and a gradient of 1:3; the diluted antibody was added to the above cells, 100 µL per well, and incubation was performed at 4°C for 1 h; after incubation, the cells were washed with the FACS buffer, 250 µL per well, centrifugation was performed at 300 g (4°C) for 5 min, and the cells were washed three times; the FACS buffer was added to prepare a secondary antibody anti-mouse IgG-Alexa488 antibody (invitrogen; A21202) (1:1k dilution), 100 µL per well, incubation was performed at 4°C in a dark place for 1 h, and then, the cells were washed again with the FACS buffer three times; and the cell pellet was resuspended with 100 µL of FACS buffer at 4°C, and the fluorescence intensity of each well of cells, i.e., binding signal, was read by a flow cytometer (BECKMAN COULTER; B53013).

### Data processing (data processing method) and experimental data:

Binding signal values corresponding to each concentration of the antibody were nonlinearly fitted by Graphpad. The data in Table 3 includes the maximum binding signal and the EC50 calculated by curve fitting of the antibody.

**Table 3 Maximum binding signal and EC50 for each antigen**

| | 293-human TSHR | | 293-monkey TSHR | | 293-mouse TSHR | |
|---|---|---|---|---|---|---|
| Antibody name | Maximum binding signal | EC50, (nM) | Maximum binding signal | EC50, (nM) | Maximum binding signal | EC50, (nM) |
| mAb001 | 21006 | 1.28 | 13564 | 0.98 | 11809 | 2.52 |
| mAb012 | 30005 | 4.74 | 24397 | 2.80 | 20750 | 8.65 |
| mAb021 | 52019 | 3.5 | 42447 | 4.5 | 31632 | 10.3 |

### Experimental conclusion:

The selected antibodies all have good binding activity to the 293 cell line overexpressing human TSHR, and all have cross-binding activity to the monkey and murine.

### Example 3: Humanized antibody cell binding experiment

Experimental operation steps:

The binding of humanized antibodies to 293-human TSHR, 293-mouse TSHR, and 293-monkey TSHR overexpressing cell lines was detected with reference to the FACS method described in Example 2. An anti-human IgG-Alexa488 antibody (invitrogen; A-11013) (1:1k dilution) was used as the secondary antibody.

### Data processing (data processing method) and experimental data:

Binding signal values corresponding to each concentration of the antibody were nonlinearly fitted by Graphpad. The data in Table 4 and Table 5 includes the maximum binding signal and the EC50 calculated by curve fitting of the antibody.

**Table 4 Maximum binding signal and EC50 for each antigen**

| Antibody name | 293-monkey TSHR | | 293-mouse TSHR | | 293-human TSHR | |
|---|---|---|---|---|---|---|
| | Maximum binding signal | EC50, (nM) | Maximum binding signal | EC50, (nM) | Maximum binding signal | EC50, (nM |
| cAb21-VH2-VL2 | 312555 | 9.3 | 175004 | 11.9 | 401580 | 10.4 |
| cAb21-VH1-VL3 | 307524 | 7.8 | 185152 | 10.4 | 397557 | 8.7 |
| cAb12-VH1-QG&S-VL3 | 269119 | 8.1 | 98806 | 14.0 | 420005 | 10.5 |
| cAb 12-VH3 - QG& S-VL3 | 302783 | 9.2 | 134651 | 15.0 | 444994 | 6.5 |

**Table 5 Maximum binding signal and EC50 for each antigen**

| Antibody name | 293-monkey TSHR | | 293-mouse TSHR | | 293-human TSHR | |
|---|---|---|---|---|---|---|
| | Maximum binding signal | EC50, (nM) | Maximum binding signal | EC50, (nM) | Maximum binding signal | EC50, (nM) |
| cAb01-VH1-DA-VL2 | 187153 | 2.19 | 113319 | 2.40 | 406930 | 3.56 |
| cAb01-VH2-DA-VL2 | 193100 | 2.14 | 117472 | 2.16 | 395619 | 3.71 |
| cAb01-VH3-DA-VL2 | 225304 | 2.29 | 128940 | 2.09 | 401397 | 3.53 |
| cAb01-VH1-DA-VL3 | 180308 | 2.30 | 105790 | 2.90 | 408761 | 3.83 |
| cAb01-VH2-DA-VL3 | 192792 | 2.60 | 122552 | 3.22 | 407873 | 3.77 |

### Experimental conclusion:

The above humanized antibodies all have good binding activity to 293-human TSHR cells, and also have the ability to bind to the monkey TSHR and the murine TSHR.

### Example 4: Humanized antibody cAMP experiment

### Experimental operation steps:

Antibody preparation: Antibodies with different concentrations were prepared in a 384-well plate (3824 Corning) by a Bravo (Agilent) instrument. The preparation solution was a DPBS with an initial concentration of 200 nM, a dilution of 1:3, and a total of 11 points.

Experiment of blocking agonistic antibody activation of cAMP by humanized antibody: According to the instruction of cAMP-Gs DYNAMIC Kit (62AM4PEB, Cisbio), antibody cAMP detection was completed. The cell line used was a 293-human TSHR cell line; competing antibodies were respectively Tab01 and Tab02, both of which were agonistic antibodies, and the concentrations used were 30 nM and 6.5 nM respectively; a sample loading instrument was MultiDrop Combi (Thermo), which was set to a low speed; and a data reading instrument was an Envision (PerkinElmer) plate reader to detect signals at wavelengths of 665 nM and 615 nM, and a standard curve was made based on the standard concentration and a signal ratio of the wavelength 665nM/615nM to detect the relative activity of the antibody.

Tab01 was a TSHR agonistic antibody M22, the sequence of which was obtained from an antibody heavy chain variable region with the sequence number No. 1 and a light chain variable region with the sequence number No. 6 in the patent WO 2004/050708A2. The heavy chain variable region and the light chain variable region were respectively linked to a human IgG1 heavy chain constant region and a human lambda light chain constant region to obtain full-length sequences of the Tab01 heavy chain and light chain, and the sequences of the human IgG1 heavy chain constant region and the human lambda light chain constant region refer to sequences of the heavy chain constant region and light chain constant region of the positive control Tab03_hIgG1.

Tab02 was a TSHR agonistic antibody K1-18, the sequence of which was obtained from a heavy chain sequence with the sequence number No. 15 and a light chain sequence with the sequence number No. 33 in the TSHR antibody patent with the patent number US10428153.

The positive control was Tab03 _hIgG1, the sequence of which was obtained from a heavy chain sequence with the sequence number No. 51 and a light chain sequence with the sequence number No. 59 in the TSHR antibody patent with the patent number US10428153.

### Data processing (data processing method) and experimental data:

The ratio of signals at wavelengths of 665 nM and 615 nM was calculated, and the ratio was directly proportional to the relative content of cAMP and represents the relative activity of TSHR. A nonlinear regression curve was drawn based on the antibody concentration and the corresponding 665 nM/615 nM ratio, and the IC50 and maximum inhibition rate were calculated. The results were shown in Table 6 and Table 7.

**Table 6 IC50 and maximum inhibition rate of each antibody**

| Antibody name | Tab01 | | Tab02 | |
|---|---|---|---|---|
| | IC50(nM) | Inhibition rate (%) | IC50(nM) | Inhibition rate (%) |
| cAb01-VH1-DA-VL2 | 1.3 | 85.58 | 0.91 | 87.39 |
| cAb01-VH2-DA-VL2 | 0.28 | 76.69 | 0.15 | 86.20 |
| cAb01-VH3-DA-VL2 | 0.29 | 83.97 | 0.23 | 82.84 |
| Tab 03_hIgG1 | 0.43 | 71.99 | 0.39 | 65.35 |

**Table 7 IC50 and maximum inhibition rate of each antibody**

| Antibody name | Tab01 | | Tab02 | |
|---|---|---|---|---|
| | IC50(nM) | Inhibition rate (%) | IC50(nM) | Inhibition rate (%) |
| cAb01-VH1-DA-VL3 | 0.085 | 82.32 | 0.054 | 88.14 |
| cAb01-VH2-DA-VL3 | 2.2 | 91.31 | 2.2 | 94.15 |
| Tab 03_hIgG1 | 0.29 | 79.03 | 0.13 | 77.05 |

### Experimental conclusion:

All antibodies show significant inhibition of TSHR activity (in the presence of agonistic antibodies Tab01 and Tab02).

### Example 5: Humanized antibody hyaluronic acid (HA) secretion experiment

### Experimental operation steps:

### 1. Acquisition of fibroblasts

The experiment was divided into three groups, and cells were respectively obtained from GO patients in the acute phase and GO patients in the stable phase. A method for acquiring cells was as follows. Orbital connective tissue specimens were obtained from patients, with 2 patients in each group. The specimens were quickly placed in sterile centrifuge tubes comprising a DMEM/F12 culture medium (comprising a mixed solution of penicillin and streptomycin), and then, the centrifuge tubes comprising the tissues were quickly transferred to a cell culture laboratory. The tissues in the centrifuge tubes were clamped in a super-clean bench and washed repeatedly with sterile PBS to remove blood stains, and then transferred to sterile culture dishes comprising a DMEM/F12 culture medium (comprising a mixed solution of penicillin and streptomycin) to ensure that the specimens were completely immersed in the culture medium to maintain the moist state and activity of the tissues. Microscopic ophthalmic scissors and tweezers were used for carefully removing adipose tissues and larger blood vessels, and the separated connective tissue was washed three times with PBS, then placed in a sterile culture dish comprising a small amount of DMEM/F12 culture medium, and cut into tissue blocks of about 1 mm³ with scissors and tweezers. The cell culture plate was rinsed once with a complete culture medium (DMEM/F12 culture medium comprising 20% of fetal bovine serum and 1% by volume of penicillin and streptomycin), and the tissue was carefully attached on the cell culture plate after being dried slightly. The cell culture plate attached with the tissue was inverted in a cell culture incubator with 5% of CO₂ at 37°C for 30 min to allow the tissue to adhere to the wall as quickly as possible. After 30 min, the cell culture plate was turned over and laid flatly, and a complete culture medium was added to just immerse the tissue for culture for several days. After 3 d, half of the medium was replaced, and floating tissue blocks and residual red blood cells were carefully removed. The crawl-out situation of cells was observed, and a culture medium was added. The cells were digested with 0.25% trypsin-EDTA every 5 to 7 days and subcultured at a ratio of 1:3 to 1:4.

### 2. Acquisition of serum immunoglobulins (GO-Igs) from GO patients

Serum samples from 40 GO patients were collected and centrifuged by a 3K15 type low-temperature high-speed centrifuge at 4°C and 12,000 rpm/min for 10 min. The serum was filtered by a filter with a filter membrane pore size of 0.45 µm and collected for later use. A gravity flow column (29920, Thermo Fisher Scientific) with a volume of 2 mL was filled with thiophillic-superflow resin (Thiophillic-Superflow, 635617, Takara) and precipitated at 4°C overnight. The affinity chromatography gravity flow column was then washed with 10 times the column volume of equilibration buffer (50 mM sodium phosphate and 0.5 M sodium sulfate, pH 7.0) for equilibration. The collected serum samples were diluted in a sample buffer (50 mM sodium phosphate and 0.55 M sodium sulfate, pH 7.0) at a ratio of 1:10 (volume ratio), and the diluted serum samples were added to the affinity chromatography gravity flow column. The sample flow rate was controlled at 2 ml/min, and then, the unbound proteins were washed with an equilibration buffer (50 mM sodium phosphate and 0.5 M sodium sulfate, pH 7.0). Finally, 2-3 times the column volume of elution buffer (20 mM sodium phosphate and 20% glycerol, pH 7.0) was used for eluting GO-Igs. The eluted products were collected and combined, and the eluted products were placed in Tube-O-Dialyzer dialysis tubes (786-619, G-Biosciences; molecular weight cutoff 50K) and dialyzed in a Hanks balanced salt solution (HBSS) comprising 10 mM HEPES (pH 7.4) at 4°C overnight. Finally, the dialyzed samples were concentrated by a Spin-X UF concentrator (CLS431480, Sigma), and the protein concentration of the concentrated product was determined by a Pierce BCA protein assay.

### 3. HA secretion experiment

### 3.1 Cell culture

The fibroblasts acquired from each group of patients were respectively counted by a Countess 3 automatic cell counter (Thermo Fisher Scientific). After adjusting the density, 5,000 or 10,000 cells were seeded into each well of a 96-well plate. The 96-well plate was placed in a Forma 3111 type water-jacketed CO₂ incubator (Thermo Electron company) and cultured overnight. All cells were observed to adhere to the wall under a microscope and then starved for 24 h, the supernatant was removed, different concentrations of the antibodies to be detected were added according to experimental groups and incubated for 24 h, and then, GO-Igs were added and further incubated for 96 h. The cell supernatant was carefully collected and centrifuged by a 3K15 type low-temperature high-speed centrifuge at 1,000×g for 15 min. The cell debris was discarded, and the supernatant was taken for detection.

### 3.2 HA detection

In this experiment, a human hyaluronic acid (HA) kit (ELISA) (ml557801, Shanghai ELISA Biotechnology Co., Ltd.) was used. The specific process was as follows: the required strips were taken out from an aluminum foil bag after equilibration at a room temperature for 60 min. Standard wells and sample wells were set, and 50 µL of standards with different concentrations were added to each standard well, wherein the concentrations were 200 ng/mL, 100 ng/mL, 50 ng/mL, 25 ng/mL, 12.5 ng/mL, and 6.25 ng/mL. 50 µL of the supernatant after the above treatment was added to the sample wells. Nothing was added to blank wells, and the wells were incubated in an incubator at 37°C for 1 h. The washing process was repeated three times with a washing solution. Except for the blank wells, 100 µL of horseradish peroxidase (HRP)-labeled detection antibody was added to each well of the standard wells and sample wells, incubated in an incubator at 37°C for 60 min, and washed five times with a washing solution. 50 µL of substrate A and 50 µL of substrate B were added to each well and incubated at 37°C in a dark place for 15 min. 50 µL of stop solution was added to each well, and the OD value of each well was measured at a wavelength of 450 nm by a microplate reader (Tecan Infinite 200 PRO microplate reader, Tecan) within 15 min.

### Data processing (data processing method) and experimental data:

A standard curve was drawn with the OD value of the measured standard as a horizontal coordinate and the concentration value of the standard as a vertical coordinate, and a linear regression equation was obtained. The OD value of the sample well was substituted into the equation to calculate the concentration of HA in each well. The HA concentration and antibody concentration were nonlinearly fitted by Graphpad prism9, and the IC50 concentration was calculated. The results were shown in Table 8.

**Table 8 Maximum inhibition rate and IC50 of each antibody**

| Antibody Name | Acute phase | | Stable phase | |
|---|---|---|---|---|
| | Maximum inhibition rate | IC50 (nM) | Maximum inhibition rate | IC50 (nM) |
| cAb21-VH2-VL2 | 64.20% | 3.00 | 74.56% | 1.15 |
| cAb21-VH1-VL3 | 65.95% | 2.55 | 76.30% | 1.93 |
| cAb01-VH1-DA-VL2 | 71.40% | 1.49 | 75.71% | 0.67 |
| cAb01-VH2-DA-VL2 | 72.09% | 2.88 | 74.79% | 1.48 |
| cAb01-VH3-DA-VL2 | 72.75% | 1.13 | 80.76% | 0.88 |
| cAb01-VH1-DA-VL3 | 70.63% | 3.28 | 76.51% | 0.67 |
| cAb01-VH2-DA-VL3 | 72.13% | 0.55 | 75.40% | 0.44 |
| cAb12- VH1-QG&S-VL3 | 73.56% | 1.63 | 77.13% | 1.19 |
| cAb12-VH3-QG&S-VL3 | 68.57% | 0.93 | 79.72% | 0.58 |
| Tab03_hIgG1 | 75.27% | 1.11 | 72.61% | 0.98 |

### Experimental conclusion:

According to the above results, it can be known that the selected antibodies all can inhibit the production of HA in orbital fibroblasts of patients stimulated by GO-Igs, wherein cAb01-VH1-DA-VL2, cAb01-VH2-DA-VL2, cAb01-VH3-DA-VL2, cAb01-VH1-DA-VL3, cAb01-VH2-DA-VL3, cAb12-VH1-QG&S-VL3, and cAb12-VH3-QG&S-VL3 have good inhibitory effects on cells in the acute phase and the stable phase, and the maximum inhibition rate in the stable phase was better than that of the control antibody Tab03_hIgG1; the IC50 of cAb01-VH2-DA-VL3 and cAb12-VH3-QG&S-VL3 was better than that of the positive control Tab03_hIgG1; and cAb21-VH2-VL2 and cAb21-VH1-VL3 have better inhibitory effects on cells in the stable phase, and the maximum inhibition rate was better than that of the Tab03_hIgG1.

### Example 6: Humanized antibody IL6 secretion experiment

### Experimental operation steps:

### 1. Cell culture

Fibroblasts and GO-Igs were collected according to the method described in Example 5, and cells were counted by a Countess 3 automatic cell counter (Thermo Fisher Scientific). After adjusting the density, 5,000 or 10,000 cells were seeded into each well of a 96-well plate. The 96-well plate was placed in a Forma 3111 type water-jacketed CO₂ incubator (Thermo Electron company) and cultured overnight. All cells were observed to adhere to the wall under a microscope and then starved for 24 h, the supernatant was removed, different concentrations of the antibodies to be detected were added according to experimental groups and incubated for 24 h, and then, GO-Igs were added and further incubated for 24 h. The cell supernatant was carefully collected and centrifuged by a 3K15 type low-temperature high-speed centrifuge at 1,000×g for 15 min. The cell debris was discarded, and the supernatant was taken for detection.

### 2. IL6 detection

In this experiment, a Human IL-6 ELISA kit (CSB-E04638h, Cusabio) was used. The specific process was as follows: the corresponding reagents were placed at a room temperature for 60 min before the experiment, 100 µl of standard and sample were added to each well, and nothing was added to the blank well. The concentrations of the standard were 500 pg/ml, 250 pg/ml, 125 pg/ml, 62.5 pg/ml, 31.2 pg/ml, 15.6 pg/ml, and 7.8 pg/ml. The plate was sealed and incubated at 37°C for 2 h. The liquid in each well was discarded, and 100 µl of 1×Biotin-antibody was added to each well and incubated in an incubator at 37°C for 1 h. The washing process was repeated three times with a washing solution. 100 µl of 1×HRP-avidin was added to each well, and the plate was sealed with a new adhesive strip and incubated at 37°C for 1 h. After washing five times, 90 µl of TMB solution was added to each well and incubated at 37°C in a dark place for 15 min. 50 µl of Stop Solution was added to each well, and the plate was gently tapped to ensure adequate mixing. Within 15 min, the OD value of each well was measured at a wavelength of 450 nm by a microplate reader (Tecan Infinite 200 PRO microplate reader, Tecan).

### Data processing (data processing method) and experimental data:

A standard curve was drawn with the OD value of the measured standard as a horizontal coordinate and the concentration value of the standard as a vertical coordinate, and a linear regression equation was obtained. The OD value of the sample was substituted into the equation to calculate the IL6 concentration in the supernatant in each well. The IL6 concentration and antibody concentration were nonlinearly fitted by Graphpad prism9, and the IC50 concentration was calculated. The results were shown in Table 9.

**Table 9 Maximum inhibition rate and IC50 of each antibody**

| Antibody Name | Acute phase | | Stable phase | |
|---|---|---|---|---|
| | Maximum inhibition rate | IC50 (nM) | Maximum inhibition rate | IC50 (nM) |
| cAb21-VH2-VL2 | 69.96% | 1.11 | 75.05% | 0.73 |
| cAb21-VH1-VL3 | 67.74% | 1.49 | 73.99% | 0.83 |
| cAb01-VH1-DA-VL2 | 71.68% | 1.34 | 75.40% | 0.85 |
| cAb01-VH2-DA-VL2 | 64.03% | 2.32 | 67.15% | 1.68 |
| cAb01-VH3-DA-VL2 | 65.98% | 1.98 | 76.61% | 0.93 |
| cAb01-VH1-DA-VL3 | 71.03% | 1.14 | 73.64% | 1.03 |
| cAb01-VH2-DA-VL3 | 69.33% | 1.06 | 73.11% | 0.60 |
| cAb12- VH1-QG&S-VL3 | 67.61% | 0.94 | 66.27% | 0.75 |
| cAb12-VH3-QG&S-VL3 | 65.91% | 2.74 | 69.73% | 1.79 |
| Tab03_hIgG1 | 69.44% | 1.51 | 67.26% | 1.00 |

### Experimental conclusion:

The selected antibodies all can inhibit the production of IL6 in orbital fibroblasts of patients stimulated by GO-Igs, and all antibodies have good inhibitory effects on cells in the acute phase and the stable phase, wherein the maximum inhibition rate and IC50 of cAb01-VH1-DA-VL2, cAb01-VH1-DA-VL3, cAb01-VH2-DA-VL3, and cAb21-VH2-VL2 for cells in the acute phase and the stable phase were better than those of the control antibody Tab03_hIgG1.

### Example 7: Humanized antibody IL8 secretion experiment

### Experimental operation steps:

### 1. Cell culture

Cell culture was performed according to the method described in Example 6, and the supernatant was collected for detection.

### 2. IL8 detection

In this experiment, a Human IL-8 ELISA kit (D8000C, R&D Company) was used for detecting the concentration of IL8 in the supernatant to be detected.

### Data processing (data processing method) and experimental data:

A standard curve was drawn with the OD value of the measured standard as a horizontal coordinate and the concentration value of the standard as a vertical coordinate, and a linear regression equation was obtained. The OD value of the sample was substituted into the equation to calculate the IL8 concentration of the supernatant in each well. The IL8 concentration and antibody concentration were nonlinearly fitted by Graphpad prism9, and the IC50 concentration was calculated.

The data analysis results were shown in Table 10.

**Table 10 Maximum inhibition rate and IC50 of each antibody**

| Antibody Name | Acute phase | | Stable phase | |
|---|---|---|---|---|
| | Maximum inhibition rate | IC50 (nM) | Maximum inhibition rate | IC50 (nM) |
| cAb21-VH2-VL2 | 69.61% | 1.87 | 78.09% | 0.55 |
| cAb21-VH1-VL3 | 68.20% | 1.87 | 72.35% | 0.59 |
| cAb01-VH1-DA-VL2 | 68.56% | 0.64 | 75.65% | 0.28 |
| cAb01-VH2-DA-VL2 | 77.28% | 0.63 | 79.49% | 0.34 |
| cAb01-VH3-DA-VL2 | 65.23% | 0.86 | 70.85% | 0.36 |
| cAb01-VH1-DA-VL3 | 65.62% | 2.35 | 61.82% | 1.93 |
| cAb01-VH2-DA-VL3 | 66.69% | 2.08 | 67.25% | 1.46 |
| cAb12- VH1-QG&S-VL3 | 67.29% | 2.21 | 74.87% | 0.69 |
| cAb12-VH3-QG&S-VL3 | 71.82% | 1.44 | 70.70% | 0.56 |
| Tab03_hIgG1 | 58.72% | 1.90 | 64.64% | 1.07 |

### Experimental conclusion:

The selected antibodies all can inhibit the production of IL8 in orbital fibroblasts of patients stimulated by GO-Igs, all antibodies have good inhibitory effects on cells in the acute phase and the stable phase, and the maximum inhibition rates of all antibodies were almost better than that of the positive control Tab03_hIgG1.

### Example 8: Engineering of humanized antibodies

In this example, the antibody cAb01-VH2-DA-VL3 (named hAb01_G4P, in which the constant region is of IgG4 subtype and comprises S228P mutation) was selected for Fc engineering (the engineering includes engineering based on IgG4-S228P and engineering based on IgG1). The purpose of the engineering is to prolong the half-life, increase the efficacy and reduce the ADCC effect. Engineered sites were shown in the following table:

**Table 11 Fc engineered sites of hAb01**

| Antibody Name | Antibody constant region subtype | Fc engineered site (EU number) |
|---|---|---|
| hAb01_G4P | hIgG4 | S228P |
| hAb01_G1_V4 | hIgG1 | L234A/L235A+M252Y/S254T/T256E |
| hAb01_G4P_V1 | hIgG4 | S228P+M252Y/S254T/T256E |
| hAb01_G4P_V2 | hIgG4 | S228P/L235E+M252Y/S254T/T256E |

### Example 9: Binding activity of engineered antibodies

The binding of engineered antibodies to 293-human TSHR, 293-mouse TSHR, and 293-monkey TSHR overexpressing cell lines was detected with reference to the FACS method described in Example 2. An anti-human IgG-Alexa488 antibody (invitrogen; A-11013) (1:1k dilution) was used as the secondary antibody.

The hIgG1 was used as a negative control (purchased from Biointron B117901); and the hIgG4 was used as a negative control (purchased from Biointron B107804).

The detection results were as follows:

**Table 12 Maximum binding signals of engineered antibodies**

| Antibody Name | 293-human TSHR | | 293-monkey TSHR | | 293-mouse TSHR | |
|---|---|---|---|---|---|---|
| | Max_MFI | EC50 (nM) | Max_MFI | EC50 (nM) | Max_MFI | EC50 (nM) |
| hAb01_G1_V4 | 371407 | 4.40 | 243621 | 2.77 | 177683 | 2.75 |
| hAb01_G4P_V1 | 281529 | 5.82 | 189666 | 2.77 | 137762 | 2.35 |
| hAb01_G4P_V2 | 287385 | 4.64 | 188885 | 2.94 | 135379 | 2.43 |
| hIgG1 | 3592 | / | 3565 | / | 2128 | / |
| hIgG4 | 1688 | / | 1767 | / | 1526 | / |

| | | | | | | |
|---|---|---|---|---|---|---|
| Note: "/" indicates a poor fit. | | | | | | |

### Experimental conclusion:

The engineered antibody binds well to the human TSHR, and has cross-binding activity to the murine and monkey.

### Example 10: cAMP activity experiment of engineered antibodies

Referring to the cAMP detection method and data processing method described in Example 4, the activity detection results of the engineered antibodies were as follows:

**Table 13 Maximum inhibition rate and IC50 of cAMP activity of engineered antibodies**

| Antibody Name | 293-human TSHR | | | |
|---|---|---|---|---|
| | Tab01 | | Tab02 | |
| | Maximum inhibition rate (%) | IC50 (nM) | Maximum inhibition rate (%) | IC50 (nM) |
| hAb01_G4P | 101.8 | 2.28 | 94.2 | 2.40 |
| hAb01 G1 V4 | 94.0 | 5.40 | 91.1 | 2.04 |
| hAb01_G4P_V1 | 105.2 | 2.95 | 100.2 | 2.74 |
| hAb01_G4P_V2 | 106.0 | 4.18 | 100.1 | 4.38 |
| hIgG1 | 9.8 | / | 4.1 | / |
| hIgG4 | 14.5 | / | 18.2 | / |

| | | | | |
|---|---|---|---|---|
| Note: "/" indicates a poor fit. | | | | |

### Experimental conclusion:

In the cAMP activity experiment, all engineered antibodies show the effect of inhibiting the activation of TSHR activity by agonistic antibodies (Tab01 and Tab02), and the inhibitory effect was not weaker than that of the nonengineered molecule hAb01_G4P.

### Example 11: ADCC activity of engineered antibodies

### Experimental method:

Target cells for the experiment were 293-human TSHR cells (the amino acid sequence of the overexpressed human TSHR was shown in P16473, Uniprot), and effector cells were human PBMCs (derived from Milestone, P122070903C). The effector cells (PBMCs) were resuscitated one day before the experiment and harvested on the day of the experiment. The cell concentration was adjusted to 5×10⁶/mL in an experimental buffer (RPMI 1640 + 10%FBS; RPMI Medium 1640, gibco, A10491-01; FBS, BI, 040021A) for later use. 1×10⁶ target cells were labeled with a fluorescent dye (Perkin Elmer, C136-100), incubated at 37°C in a dark place for 20 min, and then washed four times with PBS. The cells were resuspended in the experimental buffer for later use. The experimental buffer was used for diluting the antibody with an initial concentration of 800 nM, a gradient dilution of 1:5, and 11 points for later use. 50 µL of target cells, 100 µL of antibody diluent, and 50 µL of effector cells were added to an experimental plate. At the same time, the following control wells were set: maximum value of target cells (50 µL of target cells and 150 µL of experimental buffer); autofluorescence value of target cells (50 µL of target cells and 150 µL of experimental buffer); autofluorescence value of target cells and effector cells (50 µL of target cells, 50 µL of effector cells, and 100 µL of experimental buffer). The above experimental plate was incubated in a CO₂ incubator at 37°C for 2 h. 10 uL of lysis buffer was added to the following control wells: maximum value of target cells, maximum value of effector cells, and maximum value of target cells and effector cells. The experimental plate was centrifuged at 400 g for 5 min, 25 µL of supernatant was taken to a flat-bottomed detection plate, 200 uL of Europium Solution (Perkin Elmer, C136-100) was added to each well of the plate to be detected, and the detection plate was shaken at 250 rpm for 15 min. A reading process was performed in a multi-function microplate reader.

The sequences of the light chain variable region and heavy chain variable region of hAb01_G1 were consistent with those of hAb01_G4P, and the constant region subtype was hIgG1 (the full-length amino acid sequence of the heavy chain was set forth in SEQ ID NO: 49, and the full-length amino acid sequence of the light chain was set forth in SEQ ID NO: 51).

### Data processing (data processing method) and experimental data:

Calculation method: Inhibition rate%=(sample value - autofluorescence value of target cells and effector cells)/(maximum value of target cells - autofluorescence value of target cells)×100%.

The concentration value of the sample was used as a horizontal coordinate, and the inhibition rate was used as a vertical coordinate. The inhibition rate and antibody concentration were nonlinearly fitted by Graphpad prism9 to calculate the IC50 concentration. The results were shown in Table 14.

**Table 14 Antibody ADCC inhibition rate and IC50**

| Antibody Name | Maximum inhibition rate (%) |
|---|---|
| hIgG1 | None |
| hIgG4 | None |
| hAb01_G1 | 48.8 |
| hAb01_G4P | None |
| hAb01_G1_V4 | 30.7 |
| hAb01_G4P_V1 | None |
| hAb01_G4P_V2 | None |

| | |
|---|---|
| Note: "None" indicates no ADCC effect. | |

### Experimental conclusion:

Compared to the hAb01_G1, the hAb01_G1_V4 molecule has a weaker ADCC effect. The hAb01_G4P, hAb01_G4P_V1, and hAb01_G4P_V2 do not show an ADCC effect.

### Example 12: FcRn binding activity of engineered antibodies

Experimental method: In this experiment, an SA chip (cytiva, BR100531) was used for capturing hFcRn-Avi (Biosystems, FCM-H82W7), different concentrations of antibodies were allowed to pass through the chip, and steady-state fitting analysis was performed based on the collected data. An experimental instrument was a Biacore molecular interaction instrument (cytiva, Biacore 8K+). The buffer solution used was an HBS-EP Buffer (cytiva, BR100669). The detection was performed under the conditions that the pH was 6.0, the binding time was 240 s, the dissociation time was 240 s, the flow rate was 30 µL/min, and the temperature was 25°C. The obtained experimental data was subjected to steady-state fitting analysis by Biacore Insight Evaluation Software 3.0.12. The experimental results were shown in Table 15.

**Table 15 KD values of FcRn of engineered antibodies**

| Antibody Name | Steady-state fitting analysis | |
|---|---|---|
| | KD (M) | Response value (RU) |
| hAb01_G1 | 1.89E-07 | 60.7 |
| hAb01_G1_V4 | 9.00E-08 | 94.5 |
| hAb01_G4P | 3.43E-07 | 35.8 |
| hAb01_G4P_V1 | 1.19E-07 | 73.1 |
| hAb01_G4P_V2 | 1.21E-07 | 73.5 |

### Experimental conclusion:

Compared to antibodies of the same Fc subtype, the affinity of the engineered antibody to FcRn was significantly increased, indicating that the engineered antibody can prolong the half-life.

### Example 13: In vitro experiments of engineered antibodies for GD indications

### Experimental method:

### 1. Acquisition and culture of primary thyroid cells from patients

Thyroid tissue samples were obtained from the normal thyroid tissue of patients who underwent total thyroidectomy for thyroid cancer, and the tissue was resuspended in a PBS solution comprising 3 mg/mL of IV type collagenase (Life Technologies) to obtain dispersed cells for passage and amplification.

### 2. Thyroid peroxidase (TPO) gene level detection

The cells from each group were collected, and after adjusting the density, 6×10⁴ cells were seeded into each well of a 24-well plate and cultured overnight. After adherence, the cells were starved for 24 h, the supernatant was removed, and different concentrations of the antibodies to be detected and culture medium with or without GD serum (final concentration of 1:100, volume ratio) were added according to the experimental groups, and then, the cells were further incubated for 48 h. The cells were digested by trypsin and collected, and total RNA samples were extracted. A reverse transcription reaction system was configured according to the procedure in the kit instruction for use (iScript cDNA, Bio-Rad, 1708891EDU) to obtain cDNA which was stored at - 70°C.

### Real-time quantitative PCR reaction

The reaction system was configured on an ice box according to the procedure in the reagent instruction for use (Takara, RR420A), and amplification was performed. The data was processed by the 2-ΔΔCt formula, and the expression level of the target mRNA was obtained with the reference gene GAPDH as a reference. The primers used were shown in Table 16.

**Table 16 Primers used for real-time quantitative PCR**

| Primer | Sequence (5'→3') | |
|---|---|---|
| TPO | Positive | TGCAGTTGGCTGAGAAGAGG (SEQ ID NO: 52) |
| | Reverse | TCTGTGCAGGCCATAACCAG (SEQ ID NO: 53) |
| GAPDH | Positive | ATCATCCCTGCCTCTACTGG (SEQ ID NO: 54) |
| | Reverse | TGGGTGCGCTGTTGAAGTC (SEQ ID NO: 55) |

### Data processing and experimental data:

The data was analyzed and plotted by Graphpad Prism 9 (Version 9.4.0), the graphs were organized and combined by Adobe Illustrator 2022 (Version 2022), and the statistical differences among the groups were tested by one-way ANOVA.

The experimental results were shown in FIG. 1.

### Experimental conclusion:

All the engineered antibodies can inhibit TPO induced by serum of GD patients, indicating that the antibodies of the present invention have the potential to treat the GD disease.

### Example 14: Engineered antibody hyaluronic acid (HA) secretion experiment

The experimental method and data processing method refer to those described in Example 5.

### Data processing and experimental data:

| Antibody Name | Acute phase | | Stable phase | |
|---|---|---|---|---|
| | IC50 (nM) | Maximum concentration inhibition rate (%) | IC50 (nM) | Maximum concentration inhibition rate (%) |
| hAb01_G4_V1 | 1.39 | 82.65 | 0.88 | 82.06 |
| hAb01_G4_V2 | 1.13 | 92.22 | 0.67 | 90.97 |
| hAb01_G1_V4 | 0.45 | 87.27 | 0.75 | 84.04 |
| hIgG1 | / | 0.92 | / | 6.63 |
| hIgG4 | / | 6.99 | / | 4.70 |

| | | | | |
|---|---|---|---|---|
| Note: "/" indicates a poor fit. | | | | |

### Experimental conclusion:

According to the above results, it can be known that the engineered antibodies all can inhibit the production of HA in orbital fibroblasts of patients stimulated by GO-Igs, and have better inhibitory effects on cells in the acute phase and the stable phase.

Although the specific embodiments of the present invention have been described in detail, those skilled in the art will understand that various modifications and changes may be made to the details based on all the teachings published, and these changes are all within the scope of protection of the present invention. The entire scope of the present invention is given by the appended claims and any equivalents thereof.

## Claims

1. An antibody capable of specifically binding to a TSHR, or an antigen-binding fragment thereof, wherein the antibody or the antigen-binding fragment thereof comprises:
(a) a VH CDR1 or a variant thereof, a VH CDR2 or a variant thereof, and a VH CDR3 or a variant thereof comprised in a heavy chain variable region (VH) set forth in any one of SEQ ID NOs: 1, 27, 29, and 30; and/or a VL CDR1 or a variant thereof, a VL CDR2 or a variant thereof, and a VL CDR3 or a variant thereof comprised in a light chain variable region (VL) set forth in any one of SEQ ID NOs: 5, 28, and 31;
(b) a VH CDR1 or a variant thereof, a VH CDR2 or a variant thereof, and a VH CDR3 or a variant thereof comprised in a heavy chain variable region (VH) set forth in any one of SEQ ID NOs: 9, 32, and 34; and/or a VL CDR1 or a variant thereof, a VL CDR2 or a variant thereof, and a VL CDR3 or a variant thereof comprised in a light chain variable region (VL) set forth in SEQ ID NO: 13 or 33; or,
(c) a VH CDR1 or a variant thereof, a VH CDR2 or a variant thereof, and a VH CDR3 or a variant thereof comprised in a heavy chain variable region (VH) set forth in any one of SEQ ID NOs: 17, 35, and 37; and/or a VL CDR1 or a variant thereof, a VL CDR2 or a variant thereof, and a VL CDR3 or a variant thereof comprised in a light chain variable region (VL) set forth in any one of SEQ ID NOs: 21, 36, and 38;
wherein the variant has one or several amino acid substitutions, deletions or additions (e.g., 1, 2 or 3 amino acid substitutions, deletions or additions, such as conservative substitutions) compared to a sequence from which the variant is derived; preferably, the substitutions are conservative substitutions;
preferably, the antibody or the antigen-binding fragment thereof comprises:
(a) three CDRs comprised in a heavy chain variable region (VH) set forth in any one of SEQ ID NOs: 1, 27, 29, and 30; and/or three CDRs comprised in a light chain variable region (VL) set forth in any one of SEQ ID NOs: 5, 28, and 31;
(b) three CDRs comprised in a heavy chain variable region (VH) set forth in any one of SEQ ID NOs: 9, 32, and 34; and/or three CDRs comprised in a light chain variable region (VL) set forth in SEQ ID NO: 13 or 33; or,
(c) three CDRs comprised in a heavy chain variable region (VH) set forth in any one of SEQ ID NOs: 17, 35, and 37; and/or three CDRs comprised in a light chain variable region (VL) set forth in any one of SEQ ID NOs: 21, 36, and 38; and
preferably, the three CDRs comprised in the VH and/or the three CDRs comprised in the VL are defined by the Kabat, IMGT, or Chothia numbering system.

2. The antibody or the antigen-binding fragment thereof according to claim 1, comprising:
(a) a heavy chain variable region (VH) comprising the following three complementary determining regions (CDRs): a VH CDR1 with a sequence set forth in SEQ ID NO: 2, a VH CDR2 with a sequence set forth in SEQ ID NO: 3 or 39, and a VH CDR3 with a sequence set forth in SEQ ID NO: 4; and/or a light chain variable region (VL) comprising the following three complementary determining regions (CDRs): a VL CDR1 with a sequence set forth in SEQ ID NO: 6, a VL CDR2 with a sequence set forth in SEQ ID NO: 7, and a VL CDR3 with a sequence set forth in SEQ ID NO: 8;
(b) a heavy chain variable region (VH) comprising the following three complementary determining regions (CDRs): a VH CDR1 with a sequence set forth in SEQ ID NO: 10, a VH CDR2 with a sequence set forth in SEQ ID NO: 11 or 40, and a VH CDR3 with a sequence set forth in SEQ ID NO: 12 or 41; and/or a light chain variable region (VL) comprising the following three complementary determining regions (CDRs): a VL CDR1 with a sequence set forth in SEQ ID NO: 14, a VL CDR2 with a sequence set forth in SEQ ID NO: 15, and a VL CDR3 with a sequence set forth in SEQ ID NO: 16; or,
(c) a heavy chain variable region (VH) comprising the following three complementary determining regions (CDRs): a VH CDR1 with a sequence set forth in SEQ ID NO: 18, a VH CDR2 with a sequence set forth in SEQ ID NO: 19, and a VH CDR3 with a sequence set forth in SEQ ID NO: 20; and/or a light chain variable region (VL) comprising the following three complementary determining regions (CDRs): a VL CDR1 with a sequence set forth in SEQ ID NO: 22, a VL CDR2 with a sequence set forth in SEQ ID NO: 23, and a VL CDR3 with a sequence set forth in SEQ ID NO: 24;
wherein the CDR is defined by the Kabat numbering system.

3. The antibody or the antigen-binding fragment thereof according to claim 1 or 2, comprising:
(a) a heavy chain variable region (VH) comprising a sequence set forth in SEQ ID NO: 1 or a variant thereof; and/or a light chain variable region (VL) comprising a sequence set forth in SEQ ID NO: 5 or a variant thereof;
(b) a heavy chain variable region (VH) comprising a sequence set forth in SEQ ID NO: 9 or a variant thereof; and/or a light chain variable region (VL) comprising a sequence set forth in SEQ ID NO: 13 or a variant thereof; or,
(c) a heavy chain variable region (VH) comprising a sequence set forth in SEQ ID NO: 17 or a variant thereof; and/or a light chain variable region (VL) comprising a sequence set forth in SEQ ID NO: 21 or a variant thereof,
wherein the variant has one or several amino acid substitutions, deletions or additions (e.g., 1, 2, 3, 4, or 5 amino acid substitutions, deletions or additions), or is a sequence with at least 80%, at least 85%, at least 90%, at least 91%, at least 92%, at least 93%, at least 94%, at least 95%, at least 96%, at least 97%, at least 98%, at least 99%, or 100% sequence identity compared to a sequence from which the variant is derived; preferably, the substitutions are conservative substitutions;
preferably, the antibody or the antigen-binding fragment thereof comprises: a VH comprising a sequence set forth in SEQ ID NO: 1 and a VL comprising a sequence set forth in SEQ ID NO: 5;
preferably, the antibody or the antigen-binding fragment thereof comprises: a VH comprising a sequence set forth in SEQ ID NO: 9 and a VL comprising a sequence set forth in SEQ ID NO: 13; and
preferably, the antibody or the antigen-binding fragment thereof comprises: a VH comprising a sequence set forth in SEQ ID NO: 17 and a VL comprising a sequence set forth in SEQ ID NO: 21.

4. The antibody or the antigen-binding fragment thereof according to claim 1 or 2, wherein the antibody or the antigen-binding fragment thereof comprises a framework region sequence derived from a human immunoglobulin;
preferably, the antibody or the antigen-binding fragment thereof comprises: a heavy chain framework region of a human heavy chain germline sequence, and/or a light chain framework region of a human light chain germline sequence.

5. The antibody or the antigen-binding fragment thereof according to claim 4, wherein the antibody or the antigen-binding fragment thereof comprises:
(a) a heavy chain variable region (VH) comprising a sequence set forth in any one of SEQ ID NOs: 27, 29, and 30 or a variant thereof; and/or a light chain variable region (VL) comprising a sequence set forth in SEQ ID NO: 28 or 31 or a variant thereof;
(b) a heavy chain variable region (VH) comprising a sequence set forth in SEQ ID NO: 32 or 34 or a variant thereof; and/or a light chain variable region (VL) comprising a sequence set forth in SEQ ID NO: 33 or a variant thereof; or,
(c) a heavy chain variable region (VH) comprising a sequence set forth in SEQ ID NO: 35 or 37 or a variant thereof; and/or a light chain variable region (VL) comprising a sequence set forth in SEQ ID NO: 36 or 38 or a variant thereof,
wherein the variant has one or several amino acid substitutions, deletions or additions (e.g., 1, 2, 3, 4, or 5 amino acid substitutions, deletions or additions), or is a sequence with at least 80%, at least 85%, at least 90%, at least 91%, at least 92%, at least 93%, at least 94%, at least 95%, at least 96%, at least 97%, at least 98%, at least 99%, or 100% sequence identity compared to the sequence from which the variant is derived;
preferably, the substitutions are conservative substitutions;
preferably, the antibody or the antigen-binding fragment thereof comprises:
(a) a heavy chain variable region (VH) comprising a sequence set forth in any one of SEQ ID NOs: 27, 29, and 30; and/or a light chain variable region (VL) comprising a sequence set forth in SEQ ID NO: 28 or 31;
(b) a heavy chain variable region (VH) comprising a sequence set forth in SEQ ID NO: 32 or 34; and/or a light chain variable region (VL) comprising a sequence set forth in SEQ ID NO: 33; or,
(c) a heavy chain variable region (VH) comprising a sequence set forth in SEQ ID NO: 35 or 37; and/or a light chain variable region (VL) comprising a sequence set forth in SEQ ID NO: 36 or 38; and
preferably, the antibody or the antigen-binding fragment thereof comprises:
(1) a VH comprising a sequence set forth in SEQ ID NO: 27 and a VL comprising a sequence set forth in SEQ ID NO: 28;
(2) a VH comprising a sequence set forth in SEQ ID NO: 29 and a VL comprising a sequence set forth in SEQ ID NO: 28;
(3) a VH comprising a sequence set forth in SEQ ID NO: 30 and a VL comprising a sequence set forth in SEQ ID NO: 28;
(4) a VH comprising a sequence set forth in SEQ ID NO: 27 and a VL comprising a sequence set forth in SEQ ID NO: 31;
(5) a VH comprising a sequence set forth in SEQ ID NO: 29 and a VL comprising a sequence set forth in SEQ ID NO: 31;
(6) a VH comprising a sequence set forth in SEQ ID NO: 32 and a VL comprising a sequence set forth in SEQ ID NO: 33;
(7) a VH comprising a sequence set forth in SEQ ID NO: 34 and a VL comprising a sequence set forth in SEQ ID NO: 33;
(8) a VH comprising a sequence set forth in SEQ ID NO: 35 and a VL comprising a sequence set forth in SEQ ID NO: 36; or,
(9) a VH comprising a sequence set forth in SEQ ID NO: 37 and a VL comprising a sequence set forth in SEQ ID NO: 38.

6. The antibody or the antigen-binding fragment thereof according to any one of claims 1-5, wherein the antibody or the antigen-binding fragment thereof further comprises a constant region derived from a mammalian (e.g., human or murine) immunoglobulin;
preferably, a heavy chain of the antibody or the antigen-binding fragment thereof comprises a heavy chain constant region derived from a human immunoglobulin (e.g., IgG1, IgG2, IgG3, or IgG4), and/or a light chain of the antibody or the antigen-binding fragment thereof comprises a light chain constant region derived from a human immunoglobulin (e.g., κ or λ); and
preferably, a heavy chain of the antibody or the antigen-binding fragment thereof comprises a heavy chain constant region derived from a murine immunoglobulin (e.g., IgG1, IgG2, IgG3, or IgG4), and/or a light chain of the antibody or the antigen-binding fragment thereof comprises a light chain constant region derived from a murine immunoglobulin (e.g., κ or λ).

7. The antibody or the antigen-binding fragment thereof according to any one of claims 1-6, wherein a heavy chain of the antibody or the antigen-binding fragment thereof comprises a heavy chain constant region derived from a human immunoglobulin IgG4, and/or a light chain of the antibody or the antigen-binding fragment thereof comprises a light chain constant region derived from a human immunoglobulin (e.g., κ or λ);
preferably, compared to the heavy chain constant region of a wild type human immunoglobulin IgG4, the heavy chain constant region derived from the human immunoglobulin IgG4 comprises substitution mutations S228P, L235E, M252Y, S254T, and/or T256E (e.g., (i) S228P, (ii) L235E, and/or (iii) M252Y, S254T and T256E);
preferably, compared to the heavy chain constant region of a wild type human immunoglobulin IgG4, the heavy chain constant region derived from the human immunoglobulin IgG4 comprises a substitution mutation S228P; preferably, the heavy chain constant region derived from the human immunoglobulin IgG4 further comprises substitution mutations M252Y, S254T and T256E; and more preferably, the heavy chain constant region derived from the human immunoglobulin IgG4 further comprises substitution mutations L235E, M252Y, S254T and T256E;
preferably, a heavy chain of the antibody or the antigen-binding fragment thereof comprises a heavy chain constant region (CH) set forth in any one of SEQ ID NOs: 25 and 42-43, and/or a light chain of the antibody or the antigen-binding fragment thereof comprises a light chain constant region (CL) set forth in SEQ ID NO: 26; and
preferably, the antibody or the antigen-binding fragment thereof comprises:
(1) a heavy chain comprising a VH with a sequence set forth in SEQ ID NO: 27 and a CH with a sequence set forth in any one of SEQ ID NOs: 25 and 42-43, and/or a light chain comprising a VL with a sequence set forth in SEQ ID NO: 28 and a CL with a sequence set forth in SEQ ID NO: 26;
(2) a heavy chain comprising a VH with a sequence set forth in SEQ ID NO: 29 and a CH with a sequence set forth in any one of SEQ ID NOs: 25 and 42-43, and/or a light chain comprising a VL with a sequence set forth in SEQ ID NO: 28 and a CL with a sequence set forth in SEQ ID NO: 26;
(3) a heavy chain comprising a VH with a sequence set forth in SEQ ID NO: 30 and a CH with a sequence set forth in any one of SEQ ID NOs: 25 and 42-43, and/or a light chain comprising a VL with a sequence set forth in SEQ ID NO: 28 and a CL with a sequence set forth in SEQ ID NO: 26;
(4) a heavy chain comprising a VH with a sequence set forth in SEQ ID NO: 27 and a CH with a sequence set forth in any one of SEQ ID NOs: 25 and 42-43, and/or a light chain comprising a VL with a sequence set forth in SEQ ID NO: 31 and a CL with a sequence set forth in SEQ ID NO: 26;
(5) a heavy chain comprising a VH with a sequence set forth in SEQ ID NO: 29 and a CH with a sequence set forth in any one of SEQ ID NOs: 25 and 42-43, and/or a light chain comprising a VL with a sequence set forth in SEQ ID NO: 31 and a CL with a sequence set forth in SEQ ID NO: 26;
(6) a heavy chain comprising a VH with a sequence set forth in SEQ ID NO: 32 and a CH with a sequence set forth in any one of SEQ ID NOs: 25 and 42-43, and/or a light chain comprising a VL with a sequence set forth in SEQ ID NO: 33 and a CL with a sequence set forth in SEQ ID NO: 26;
(7) a heavy chain comprising a VH with a sequence set forth in SEQ ID NO: 34 and a CH with a sequence set forth in any one of SEQ ID NOs: 25 and 42-43, and/or a light chain comprising a VL with a sequence set forth in SEQ ID NO: 33 and a CL with a sequence set forth in SEQ ID NO: 26;
(8) a heavy chain comprising a VH with a sequence set forth in SEQ ID NO: 35 and a CH with a sequence set forth in any one of SEQ ID NOs: 25 and 42-43, and/or a light chain comprising a VL with a sequence set forth in SEQ ID NO: 36 and a CL with a sequence set forth in SEQ ID NO: 26; or,
(9) a heavy chain comprising a VH with a sequence set forth in SEQ ID NO: 37 and a CH with a sequence set forth in any one of SEQ ID NOs: 25 and 42-43, and/or a light chain comprising a VL with a sequence set forth in SEQ ID NO: 38 and a CL with a sequence set forth in SEQ ID NO: 26.

8. The antibody or the antigen-binding fragment thereof according to any one of claims 1-6, wherein a heavy chain of the antibody or the antigen-binding fragment thereof comprises a heavy chain constant region derived from a human immunoglobulin IgG1, and/or a light chain of the antibody or the antigen-binding fragment thereof comprises a light chain constant region derived from a human immunoglobulin (e.g., κ or λ);
preferably, compared to the heavy chain constant region of a wild type human immunoglobulin IgG1, the heavy chain constant region derived from the human immunoglobulin IgG1 comprises substitution mutations L234A, L235A, M252Y, S254T, and/or T256E (e.g., (i) L234A and L235A, and/or (ii) M252Y, S254T and T256E);
preferably, compared to the heavy chain constant region of a wild type human immunoglobulin IgG1, the heavy chain constant region derived from the human immunoglobulin IgG1 comprises substitution mutations L234A and L235A; preferably, the heavy chain constant region derived from the human immunoglobulin IgG1 further comprises substitution mutations M252Y, S254T and T256E;
preferably, a heavy chain of the antibody or the antigen-binding fragment thereof comprises a heavy chain constant region (CH) set forth in SEQ ID NO: 44 or 45, and/or a light chain of the antibody or the antigen-binding fragment thereof comprises a light chain constant region (CL) set forth in SEQ ID NO: 26; and
preferably, the antibody or the antigen-binding fragment thereof comprises:
(1) a heavy chain comprising a VH with a sequence set forth in SEQ ID NO: 27 and a CH with a sequence set forth in SEQ ID NO: 44 or 45, and/or a light chain comprising a VL with a sequence set forth in SEQ ID NO: 28 and a CL with a sequence set forth in SEQ ID NO: 26;
(2) a heavy chain comprising a VH with a sequence set forth in SEQ ID NO: 29 and a CH with a sequence set forth in SEQ ID NO: 44 or 45, and/or a light chain comprising a VL with a sequence set forth in SEQ ID NO: 28 and a CL with a sequence set forth in SEQ ID NO: 26;
(3) a heavy chain comprising a VH with a sequence set forth in SEQ ID NO: 30 and a CH with a sequence set forth in SEQ ID NO: 44 or 45, and/or a light chain comprising a VL with a sequence set forth in SEQ ID NO: 28 and a CL with a sequence set forth in SEQ ID NO: 26;
(4) a heavy chain comprising a VH with a sequence set forth in SEQ ID NO: 27 and a CH with a sequence set forth in SEQ ID NO: 44 or 45, and/or a light chain comprising a VL with a sequence set forth in SEQ ID NO: 31 and a CL with a sequence set forth in SEQ ID NO: 26;
(5) a heavy chain comprising a VH with a sequence set forth in SEQ ID NO: 29 and a CH with a sequence set forth in SEQ ID NO: 44 or 45, and/or a light chain comprising a VL with a sequence set forth in SEQ ID NO: 31 and a CL with a sequence set forth in SEQ ID NO: 26;
(6) a heavy chain comprising a VH with a sequence set forth in SEQ ID NO: 32 and a CH with a sequence set forth in SEQ ID NO: 44 or 45, and/or a light chain comprising a VL with a sequence set forth in SEQ ID NO: 33 and a CL with a sequence set forth in SEQ ID NO: 26;
(7) a heavy chain comprising a VH with a sequence set forth in SEQ ID NO: 34 and a CH with a sequence set forth in SEQ ID NO: 44 or 45, and/or a light chain comprising a VL with a sequence set forth in SEQ ID NO: 33 and a CL with a sequence set forth in SEQ ID NO: 26;
(8) a heavy chain comprising a VH with a sequence set forth in SEQ ID NO: 35 and a CH with a sequence set forth in SEQ ID NO: 44 or 45, and/or a light chain comprising a VL with a sequence set forth in SEQ ID NO: 36 and a CL with a sequence set forth in SEQ ID NO: 26; or,
(9) a heavy chain comprising a VH with a sequence set forth in SEQ ID NO: 37 and a CH with a sequence set forth in SEQ ID NO: 44 or 45, and/or a light chain comprising a VL with a sequence set forth in SEQ ID NO: 38 and a CL with a sequence set forth in SEQ ID NO: 26.

9. The antibody or the antigen-binding fragment thereof according to any one of claims 1-8, wherein the antigen-binding fragment is selected from Fab, Fab', (Fab')₂, Fd, Fv, disulfide-linked Fv, scFv, di-scFv, (scFv)₂, a diabody, and a single domain antibody (sdAb); and/or the antibody is a murine antibody, a humanized antibody, a chimeric antibody, a bispecific antibody, or a multispecific antibody.

10. An isolated nucleic acid molecule encoding the antibody or the antigen-binding fragment thereof according to any one of claims 1-9, or the heavy chain variable region and/or the light chain variable region thereof, or the heavy chain and/or the light chain thereof.

11. A vector, comprising the isolated nucleic acid molecule according to claim 10, preferably, the vector is a cloning vector or an expression vector.

12. A host cell, comprising the isolated nucleic acid molecule according to claim 10 or the vector according to claim 11.

13. A method for preparing the antibody or the antigen-binding fragment thereof according to any one of claims 1-9, comprising: culturing the host cell of claim 12 under the conditions allowing the expression of the antibody or the antigen-binding fragment thereof, and recovering the antibody or the antigen-binding fragment thereof from the cultured host cell culture.

14. A bispecific or multispecific molecule, comprising the antibody or the antigen-binding fragment thereof according to any one of claims 1-9, wherein
preferably, the bispecific or multispecific molecule specifically binds to a TSHR, and additionally specifically binds to one or more other targets;
preferably, the bispecific or multispecific molecule further comprises at least one molecule (e.g., a second antibody) with a second binding specificity for a second target;
preferably, the bispecific or multispecific molecule further comprises at least one second antibody that specifically binds to an IGF1R; and preferably, the second antibody has antagonism on IGF1R signaling.

15. An immunoconjugate, comprising the antibody or the antigen-binding fragment thereof according to any one of claims 1-9 and a therapeutic agent linked to the antibody or the antigen-binding fragment thereof, wherein
preferably, the therapeutic agent is selected from an IGF1R antagonist or inhibitor; and
preferably, the immunoconjugate is an antibody-drug conjugate (ADC).

16. A pharmaceutical composition, comprising the antibody or the antigen-binding fragment thereof according to any one of claims 1-9, the isolated nucleic acid molecule according to claim 10, the vector according to claim 11, the host cell according to claim 12, the bispecific or multispecific molecule according to claim 14 or the immunoconjugate according to claim 15, as well as a pharmaceutically acceptable carrier and/or excipient, wherein
preferably, the pharmaceutical composition further comprises another pharmaceutically active agent, such as another drug for treating an autoimmune thyroid disease (e.g., Graves' disease or Graves' ophthalmopathy) or thyroid cancer; and
preferably, the pharmaceutical composition further comprises an IGF1R antagonist or inhibitor, and/or another TSHR antagonist or inhibitor.

17. Use of the antibody or the antigen-binding fragment thereof according to any one of claims 1-9, the isolated nucleic acid molecule according to claim 10, the vector according to claim 11, the host cell according to claim 12, the bispecific or multispecific molecule according to claim 14, the immunoconjugate according to claim 15 or the pharmaceutical composition according to claim 16 for preparing a drug, wherein the drug is used for preventing and/or treating a TSHR-related disease in a subject;
preferably, the TSHR-related disease benefits from the antagonism on TSHR signaling;
preferably, the TSHR-related disease is an autoimmune thyroid disease (e.g., Graves' disease or Graves' ophthalmopathy), thyroid cancer, or a combination thereof;
preferably, the subject is a mammal, such as a human, murine, or monkey;
preferably, the antibody or the antigen-binding fragment thereof, the isolated nucleic acid molecule, the vector, the host cell, the bispecific or multispecific molecule, the immunoconjugate, or the pharmaceutical composition is used alone or in combination with another pharmaceutically active agent (e.g., another drug for treating an autoimmune thyroid disease (e.g., Graves' disease or Graves' ophthalmopathy) or thyroid cancer); and
preferably, the antibody or the antigen-binding fragment thereof, the isolated nucleic acid molecule, the vector, the host cell, the bispecific or multispecific molecule, the immunoconjugate, or the pharmaceutical composition is used in combination with an IGF1R antagonist or inhibitor and/or another TSHR antagonist or inhibitor.

18. A method for preventing and/or treating a TSHR-related disease in a subject, comprising: administering to a subject in need thereof an effective amount of the antibody or the antigen-binding fragment thereof according to any one of claims 1-9, the isolated nucleic acid molecule according to claim 10, the vector according to claim 11, the host cell according to claim 12, the bispecific or multispecific molecule according to claim 14, the immunoconjugate according to claim 15, or the pharmaceutical composition according to claim 16;
preferably, the TSHR-related disease benefits from the antagonism on TSHR signaling;
preferably, the TSHR-related disease is an autoimmune thyroid disease (e.g., Graves' disease or Graves' ophthalmopathy), thyroid cancer, or a combination thereof;
preferably, the subject is a mammal, such as a human, murine, or monkey;
preferably, the antibody or the antigen-binding fragment thereof, the isolated nucleic acid molecule, the vector, the host cell, the bispecific or multispecific molecule, the immunoconjugate, or the pharmaceutical composition is used alone or in combination with another pharmaceutically active agent (e.g., another drug for treating an autoimmune thyroid disease (e.g., Graves' disease or Graves' ophthalmopathy) or thyroid cancer); and
preferably, the antibody or the antigen-binding fragment thereof, the isolated nucleic acid molecule, the vector, the host cell, the bispecific or multispecific molecule, the immunoconjugate, or the pharmaceutical composition is used in combination with an IGF1R antagonist or inhibitor and/or another TSHR antagonist or inhibitor.

19. A conjugate, comprising the antibody or the antigen-binding fragment thereof according to any one of claims 1-9 and a detectable label linked to the antibody or the antigen-binding fragment thereof, wherein
preferably, the detectable label is selected from an enzyme (e.g., horseradish peroxidase or alkaline phosphatase), a chemiluminescent reagent (e.g., acridinium ester compounds, luminol and derivatives thereof, or ruthenium derivatives), a fluorescent dye (e.g., fluorescein or fluorescent protein), a radionuclide, or a biotin.

20. A kit, comprising the antibody or the antigen-binding fragment thereof according to any one of claims 1-9 or the conjugate according to claim 19.

21. A method for detecting the presence or level of a TSHR in a sample, comprising using the antibody or the antigen-binding fragment thereof according to any one of claims 1-9 or the conjugate according to claim 19;
preferably, the method is an immunological assay, such as an immunoblot, an enzyme immunoassay (e.g., ELISA), a chemiluminescent immunoassay, a fluorescent immunoassay, or a radioimmunoassay;
preferably, the method comprises using the conjugate according to claim 19;
preferably, the method comprises using the antibody or the antigen-binding fragment thereof according to any one of claims 1-9, and the method also includes using a second antibody carrying a detectable label (e.g., an enzyme (e.g., horseradish peroxidase or alkaline phosphatase), a chemiluminescent reagent (e.g., acridinium ester compounds, luminol and derivatives thereof, or ruthenium derivatives), a fluorescent dye (e.g., fluorescein or fluorescent protein), a radionuclide, or a biotin) to detect the antibody or the antigen-binding fragment thereof;
preferably, the method comprises: (1) contacting the sample with the antibody or the antigen-binding fragment thereof according to any one of claims 1-9 or the conjugate according to claim 19; and (2) detecting the formation of an antigen-antibody immune complex or detecting the amount of the immune complex, wherein the formation of the immune complex indicates the presence of the TSHR or cells expressing the TSHR;
preferably, the method is one for diagnosing a TSHR-related disease, wherein the sample is one from a subject;
preferably, the presence of the TSHR or an increased level of the TSHR compared to a reference level (e.g., compared to a healthy control) indicates that the subject suffers from a TSHR-related disease; and
preferably, the TSHR-related disease is an autoimmune thyroid disease (e.g., Graves' disease or Graves' ophthalmopathy), thyroid cancer, or a combination thereof.

22. Use of the antibody or the antigen-binding fragment thereof according to any one of claims 1-9 or the conjugate according to claim 19 in the preparation of a detection reagent, wherein the detection reagent is used for detecting the presence or level of a TSHR in a sample and/or diagnosing a TSHR-related disease;
preferably, the detection reagent is used to detect the presence or level of the TSHR in a sample or diagnose a TSHR-related disease by the method according to claim 21;
preferably, the TSHR-related disease is an autoimmune thyroid disease (e.g., Graves' disease or Graves' ophthalmopathy), thyroid cancer, or a combination thereof; and
preferably, the sample is a tissue sample from a subject (e.g., a mammal, preferably a human, murine, or monkey).
